# EUROPEAN PATENT APPLICATION

(11) **EP 4 541 267 A1**
(43) Date of publication of application: **23.04.2025**
(21) Application number: 24728003.5
(22) Date of filing: 22.05.2024
(51) Int. Cl.: A61B 5/00, A61B 5/01

(54) **APPARATUS AND METHOD FOR GUIDING WEARING OF WEARABLE DEVICE**

(30) Priority: 07.09.2023 KR 20230118811; 18.10.2023 KR 20230139916
(71) Applicant: Samsung Electronics Co., Ltd., Suwon-si, Gyeonggi-do 16677 (KR)
(72) Inventor: LEE, Taehan, Yeongtong-gu, Suwon-si Gyeonggi-do 16677 (KR); KANG, Junghyun, Yeongtong-gu, Suwon-si Gyeonggi-do 16677 (KR); LEE, June, Yeongtong-gu, Suwon-si Gyeonggi-do 16677 (KR)
(74) Representative: Gulde & Partner
(86) International application number: PCT/KR2024/006912
(87) International publication number: WO 2025/053372

(57) **Abstract**

A wearable device includes memory storing instructions, a first sensor and a second sensor, a display, and at least one processor. The instructions, when executed by the at least one processor individually or collectively, cause the wearable device to obtain, by using the first sensor, first data on pressure between the wearable device and a body portion of a user on which the wearable device is worn, obtain, by using the second sensor, second data used for measuring at least one of a temperature, a humidity, or active oxygen of the body portion, decide whether a probability of damage of a skin of the body portion is happened based on a length of time when the wearable device is worn on the body portion, the first data, and the second data, and display a visual object to guide taking off the wearable device from the body portion based on the decision that the probability of damage portion is happened.

## Description

### [Technical Field]

Descriptions below relate to a device and a method for guiding wearing of a wearable device.

### [Background Art]

An electronic device may include a wearable device that may be worn by a user. For example, the wearable device may be worn on a body portion of the user. For example, the body portion may include an ear portion or a wrist portion of the user.

The wearable device may include at least one sensor. For example, the wearable device may obtain data by using the at least one sensor.

The above-described information may be provided as a related art for the purpose of helping to understand the present disclosure. No claim or determination is raised as to whether any of the above-described information may be applied as a prior art related to the present disclosure.

### [Disclosure]

### [Technical Solution]

The present invention is defined by the appended set of claims. Further embodiment serves illustrative and comparative purpose.

A wearable device may comprise a plurality of sensors including a first sensor and a second sensor. The wearable device may comprise a display. The wearable device may comprise at least one processor. The at least one processor may be configured to obtain, by using the first sensor, first data related to pressure between the wearable device and a body portion of a user on which the wearable device is worn. The at least one processor may be configured to obtain, by using the second sensor, a second data used for measuring at least one of a temperature, a humidity, or active oxygen of the body portion while the wearable device is worn on the body portion. The at least one processor may be configured to decide whether a probability of damage of a skin of the body portion is happened based on a length of time when the wearable device is worn on the body portion, the first data, and the second data. The at least one processor may be configured to display, through the display, a visual object to guide taking off the wearable device from the body portion based on the decision that the probability of damage portion is happened.

A wearable device may comprise a plurality of sensors including a first sensor and a second sensor. The wearable device may comprise a speaker. The wearable device may comprise at least one processor. The at least one processor may be configured to obtain, by using the first sensor, first data related to movement of the wearable device based on recognizing that the wearable device is worn on a body portion of a user. The at least one processor may be configured to obtain, by using the second sensor, a second data used for measuring at least one of a temperature, a humidity, or active oxygen of the body portion while the wearable device is worn on the body portion. The at least one processor may be configured to decide whether a probability of damage of a skin of the body portion is happened based on a length of time when the wearable device is worn on the body portion, the first data, and the second data. The at least one processor may be configured to provide, through the speaker, a notification to guide taking off the wearable device from the body portion based on the decision that the probability of damage is happened.

In a method performed by a wearable device, the method may comprise obtaining first data related to pressure between the wearable device and a body portion of a user on which the wearable device is worn. The method may comprise obtaining a second data used for measuring at least one of a temperature, a humidity, or active oxygen of the body portion while the wearable device is worn on the body portion. The method may comprise deciding whether a probability of damage to a skin of the body portion is happened based on a length of time when the wearable device is worn on the body portion, the first data, and the second data. The method may comprise displaying a visual object to guide taking off the wearable device from the body portion based on the decision that the probability of damage is happed.

A method performed by a wearable device may comprise obtaining first data related to movement of the wearable device based on recognizing that the wearable device is worn on a body portion of a user. The method may comprise obtaining second data used for measuring at least one of a temperature, a humidity, or active oxygen of the body portion while the wearable device is worn on the body portion. The method may comprise deciding whether a probability of damage to a skin of the body portion is happened based on a length of time when the wearable device is worn on the body portion, the first data, and the second data. The method may comprise providing a notification to guide taking off the wearable device from the body portion based on the decision that the probability of damage is happed.

A non-transitory computer-readable storage medium, when individually or collectively executed by at least one processor of a wearable device comprising a plurality of sensors including a first sensor and a second sensor and a display, may store one or more programs including instructions that cause to obtain, by using the first sensor, first data related pressure between the wearable device and a body portion of a user on which the wearable device is worn. The non-transitory computer-readable storage medium, when individually or collectively executed by the at least one processor, may store one or more programs including instructions that cause to obtain, by using the second sensor, second data used for measuring at least one of a temperature, a humidity, or active oxygen of the body portion while the wearable device is worn on the body portion. The non-transitory computer-readable storage medium, when individually or collectively executed by the at least one processor, may store one or more programs including instructions that cause to decide whether a probability of damage of a skin of the body portion is happened based on a length of time when the wearable device is worn on the body portion, the first data, and the second data. The non-transitory computer-readable storage medium, when individually or collectively executed by the at least one processor, may store one or more programs including instructions that cause to display, through the display, a visual object to guide taking off the wearable device from the body portion based on the decision that the probability of damage is happened.

A non-transitory computer-readable storage medium, when individually or collectively executed by at least one processor of a wearable device comprising a plurality of sensors including a first sensor and a second sensor and a speaker, may store one or more programs including instructions that cause to obtain, by using the first sensor, first data related to movement of the wearable device based on recognizing that the wearable device is worn on a body portion of a user. The non-transitory computer-readable storage medium, when individually or collectively executed by the at least one processor, may store one or more programs including instructions that cause to obtain, by using the second sensor, a second data used for measuring at least one of a temperature, a humidity, or active oxygen of the body portion while the wearable device is worn on the body portion. The non-transitory computer-readable storage medium, when individually or collectively executed by the at least one processor, may store one or more programs including instructions that cause to decide whether a probability of damage of a skin of the body portion is happened based on a length of time when the wearable device is worn on the body portion, the first data, and the second data. The non-transitory computer-readable storage medium, when individually or collectively executed by the at least one processor, may store one or more programs including instructions that cause to provide, through the speaker, a notification to guide taking off the wearable device from the body portion based on the decision that the probability of damage is happened.

### [Description of the Drawings]

FIG. 1 is a block diagram of an electronic device in a network environment, according to various embodiments.
FIG. 2 illustrates an example of a method of recognizing damage to a body portion of a user and guiding a wearing method according to wearing of a wearable device.
FIG. 3A illustrates an exemplary block diagram of a wearable device worn on a wrist portion of a user.
FIGS. 3B and 3C illustrate examples of components included in a wearable device worn on a wrist portion of a user.
FIGS. 4A and 4B illustrate examples of a first sensor included in a wearable device.
FIG. 5A illustrates an exemplary block diagram of a wearable device worn on an ear portion of a user.
FIG. 5B illustrates an example of components included in a wearable device worn on an ear portion of a user.
FIG. 6A illustrates an example of an operation flow of a method of recognizing the degree of damage to a body portion on which a wearable device is worn and displaying a visual object guiding a wearing method.
FIG. 6B illustrates an example of a visual object guiding a wearing method of a wearable device.
FIG. 7A illustrates an example of an operation flow of a method in which a wearable device worn on a wrist portion of a user guides a wearing method.
FIGS. 7B to 7D illustrate examples of a visual object guiding a wearing method of a wearable device.
FIG. 8A illustrates an example of an operation flow of a method of recognizing the degree of damage to a body portion on which a wearable device is worn and providing a notification for guiding a wearing method.
FIG. 8B illustrates an example of a method of displaying a visual object guiding a wearing method of a wearable device through an external electronic device.
FIG. 9A illustrates an example of an operation flow of a method in which a wearable device worn on an ear portion of a user guides a wearing method.
FIG. 9B illustrates an example of a method of displaying a visual object guiding a normal wearing state of a wearable device through an external electronic device.

### [Mode for Invention]

Terms used in the present disclosure are used only to describe a specific embodiment, and may not be intended to limit the scope of another embodiment. A singular expression may include a plural expression unless it is clearly meant differently in the context. The terms used herein, including a technical or scientific term, may have the same meaning as generally understood by a person having ordinary knowledge in the technical field described in the present disclosure. Terms defined in a general dictionary among the terms used in the present disclosure may be interpreted with the same or similar meaning as a contextual meaning of related technology, and unless clearly defined in the present disclosure, it is not interpreted in an ideal or excessively formal meaning. In some cases, even terms defined in the present disclosure cannot be interpreted to exclude embodiments of the present disclosure.

In various embodiments of the present disclosure described below, a hardware approach is described as an example. However, since the various embodiments of the present disclosure include technology that use both hardware and software, the various embodiments of the present disclosure do not exclude a software-based approach.

In addition, in the present disclosure, in order to determine whether a specific condition is satisfied or fulfilled, an expression of more than or less than may be used, but this is only a description for expressing an example, and does not exclude description of more than or equal to or less than or equal to. A condition described as 'more than or equal to' may be replaced with ' more than', a condition described as 'less than or equal to' may be replaced with 'less than', and a condition described as 'more than or equal to and less than' may be replaced with 'more than and less than or equal to'. In addition, hereinafter, 'A' to 'B' means at least one of elements from A (including A) and to B (including B).

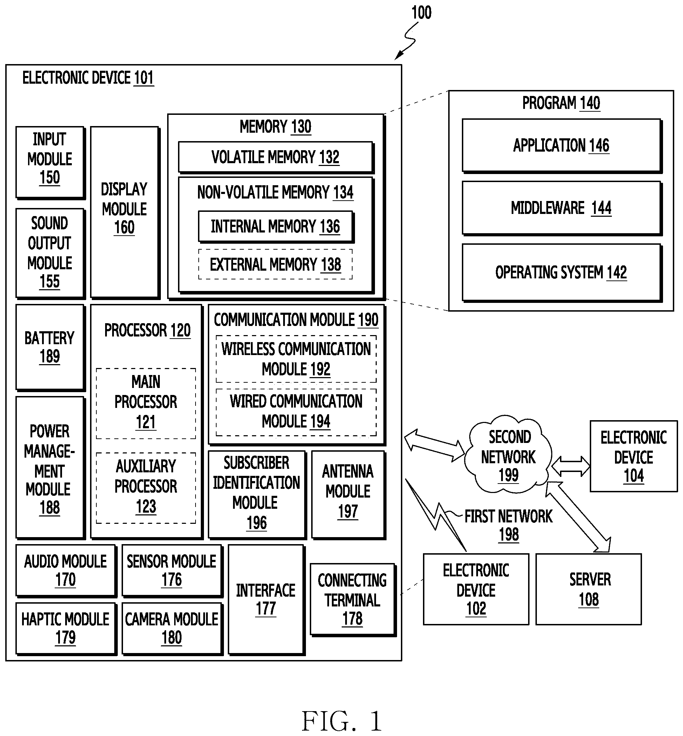

FIG. 1 illustrates a block diagram of an electronic device in a network environment according to various embodiments.

Referring to FIG. 1, an electronic device 101 in a network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, a memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in a volatile memory 132, process the command or the data stored in the volatile memory 132, and store resulting data in a non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high-definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mm Wave band) to address, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large-scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element including a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra-low-latency services using, e.g., distributed computing or mobile edge computing. In another example of the disclosure, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

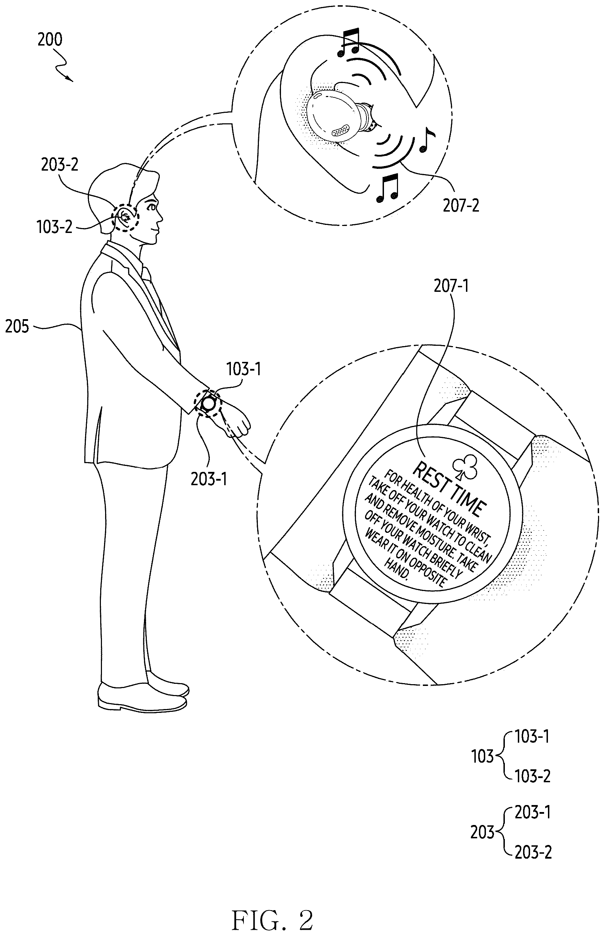

FIG. 2 illustrates an example of a method of recognizing damage to a body portion of a user and guiding a wearing method according to wearing of a wearable device.

FIG. 2 illustrates an example 200 of a method of recognizing damage to a body portion 203 of a user 205 and guiding a wearing method according to wearing of a wearable device 103. The wearable device 103 of FIG. 2 may indicate an example of the electronic device 102 of FIG. 1. Although not illustrated in the example 200 of FIG. 2, the wearable device 103 may be connected to an electronic device 101 of FIG. 1.

For example, the wearable device 103 may include a wearable device 103-1 worn on a wrist portion 203-1 of the user 205. For example, the wearable device 103 may include a wearable device 103-2 worn on an ear portion 203-2 of the user 205. However, the embodiment of the present disclosure is not limited thereto. For example, the wearable device 103 may include a ringshaped wearable device worn on a finger portion of the user 205. For example, in a state of being connected to the electronic device 101 of FIG. 1, the wearable device 103 may receive a signal from the electronic device 101 and may provide content based on the signal. For example, in the above state, the wearable device 103-1 may display a visual object through a display of the wearable device 103-1 in response to an event identified by the electronic device 101. For example, the wearable device 103-2 may output sound information obtained from the electronic device 101 in the above state through an output device (e.g., a speaker) of the wearable device 103-2. However, the embodiment of the present disclosure is not limited thereto.

Referring to the example 200, the wearable device 103-1 may be worn on the wrist portion 203-1 of the user 205. For example, the wearable device 103-1 may have a watch shape. For example, the wearable device 103-1 may be referred to as a watch or a smart watch. For example, the wrist portion 203-1 on which the wearable device 103-1 is worn may contact at least a portion of the wearable device 103-1. For example, the at least portion may include a portion (e.g., a plate or a rear plate) of a housing of the wearable device 103-1 and a strap (or a band) included in the wearable device 103-1. A skin of the wrist portion 203-1 contacting the wearable device 103-1 may be damaged. For example, in case that the wearable device 103-1 is worn for a long time or the wearable device 103-1 is in tight contact with the wrist portion 203-1, the skin of the wrist portion 203-1 may be damaged.

According to an embodiment, the wearable device 103-1 may recognize a condition (or degree of damage) of the skin and may display a visual object 207-1. For example, the visual object 207-1 may display a visual object that guides taking off (or release) (or taking off wearing) a fastening state of the wearable device 103-1. However, the embodiment of the present disclosure is not limited thereto. For example, the visual object 207-1 may include a visual object that guides the fastening state of the wearable device 103-1 to change from a tight state to a loose state. As the visual object 207-1 displayed on the wearable device 103-1 is recognized, the user 205 may take off or loosely wear the wearable device 103-1.

In addition, referring to the example 200, the wearable device 103-2 may be worn on the ear portion 203-2 of the user 205. For example, the wearable device 103-2 may be referred to as earbuds, earphone, or true wireless stereo (TWS). For example, the ear portion 203-2 on which the wearable device 103-2 is worn may contact at least a portion of the wearable device 103-2. For example, the at least portion may include a portion of a housing of the wearable device 103-2 and a portion of an eartip included in the wearable device 103-2. For example, the eartip may be connected to a nozzle of the wearable device 103-2. A skin of the ear portion 203-2 contacting the wearable device 103-2 may be damaged. For example, in case of wearing the wearable device 103-2 for a long time, the skin of the ear portion 203-2 may be damaged.

According to an embodiment, the wearable device 103-2 may recognize the condition (or degree of damage) of the skin and may output sound information 207-2. For example, the sound information 207-2 may include sound for text that guides taking off (or release) (or taking off wearing) a wearing state of the wearable device 103-2. However, the embodiment of the present disclosure is not limited thereto. As the sound information 207-2 outputted from the wearable device 103-2 is recognized, the user 205 may take off the wearable device 103-2.

Referring to those described above, embodiments of the present disclosure may guide the wearing of the wearable device 103 through a sensor capable of detecting the wearing state of the wearable device 103-1 worn on the wrist such as the smart watch or the wearing state of the wearable device 103-2 worn on the ear such as the TWS and the earphone and a skin state (or condition) of the body portion on which the wearable device 103 is worn.

Irritant contact dermatitis (ICD) is an inflammatory skin disease caused by skin barrier destruction or damage due to an exogenous factor or an environmental factor along with activation of innate immune response. For example, the irritant contact dermatitis (ICD) may be caused by wet work, chemicals (soap, detergents, solvents, and oils), friction pressure and vibration, heat, cold, humidity, and ultraviolet rays. In addition, otitis externa is a symptom that inflammation is caused by being infected by bacteria and fungi. In case of using canal-type earphones or TWS, since moisture or sweat occurs and it is warm, it is a good environment for the bacteria or the fungi to reproduce, so the otitis externa may occur.

For example, an electronic device using a sensor for detecting pressure may guide a wearing state of a band (or a strap) of the electronic device. However, the electronic device cannot guide by detecting a wearing state (i.e., an abnormal wearing state) in which unbalanced pressure exists, or detecting an environment and a wearing state in which wound, rash, inflammation, burn, and the like may occur on the body portion (e.g., the wrist portion) where the electronic device is worn. In addition, for example, as a method of measuring hydration of the skin, the electronic device may measure the hydration of the skin through the sensor of the strap. However, it may be restricted to recognize whether the electronic device is the environment in which the wound, the rash, the inflammation, and the burn of the skin may occur, by only measuring the hydration of the skin.

In case of the wearable device 103-1, it is recommended to wear it tightly so that the sensor of the wrist portion and the wrist portion may be in contact with each other to obtain data for health care. As the wearable device 103-1 is worn for a long time in a sweaty state after exercise and without ventilation, the rash is likely to occur on the wrist. Thus, a method for guiding wearing and wearing time of the wearable device 103-1 based on an appropriate level of pressure is required. In addition, in case of the wearable device 103-2, it is recommended to avoid wearing it for a long time so that it does not become an environment in which the bacteria and the fungi may reproduce. However, since the inflammation may occur even for a relatively short period of time according to a surrounding environment and a state (e.g., temperature or humidity, presence of wound) of inside (or the ear portion) of an ear canal, a method for guiding an appropriate level of wearing time of the wearable device 103-2 is required.

The embodiments of the present disclosure may guide a wearing method of the wearable device 103 by recognizing the wearing state of the wearable device 103 and using a sensor for detecting the skin state (or condition).

For example, the wearable device 103-1 may use at least one sensor to recognize the wearing state (e.g., pressure). For example, the at least one sensor for recognizing the wearing state may include a force sensor, a strain sensor, and a barometer. The wearable device 103-1 may recognize the wearing state based on the pressure measured based on the at least one sensor, or may recognize a balance of pressure based on an array of a plurality of sensors. In addition, for example, the wearable device 103-2 may recognize the wearing state based on reflection of sound (e.g., chirp or click) for the entire frequency, a sound difference between microphones (e.g., an internal microphone and an external microphone) of the wearable device 103-2, or a level of physical shielding of the sound played by the speaker. Alternatively, for example, the wearable device 103-2 may recognize the wearing state based on an impedance size measured based on a conductive nozzle (or eartip).

For example, the wearable device 103 may use at least one other sensor to recognize the skin state (or condition). For example, the at least one other sensor may include a sensor for measuring the sweat of the skin, a sensor for measuring the humidity of the skin, a sensor for measuring the temperature of the skin, or a sensor for measuring oxygen information (e.g., antioxidant degree or active oxygen) of the skin. For example, the oxygen information may be used to recognize whether the inflammation has occurred on the skin, based on amount of change in the antioxidant degree or the active oxygen of the skin.

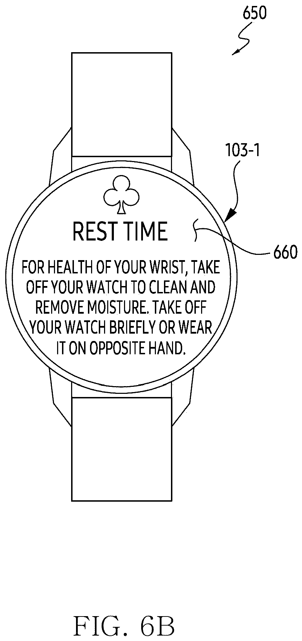

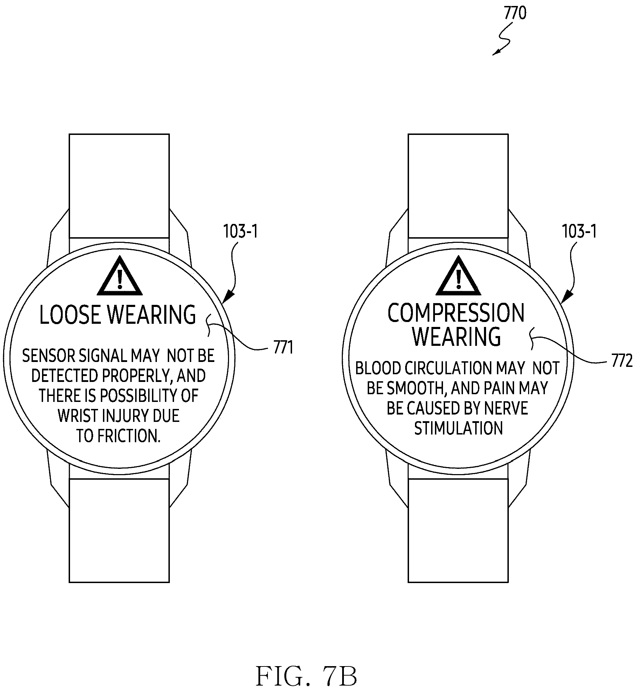

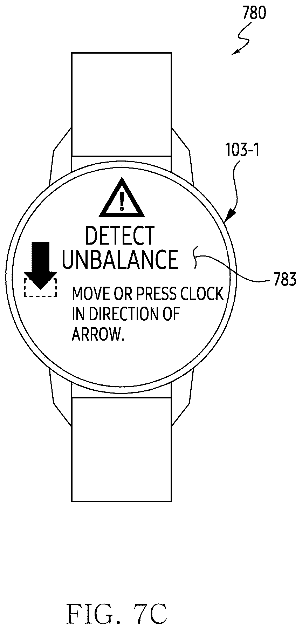

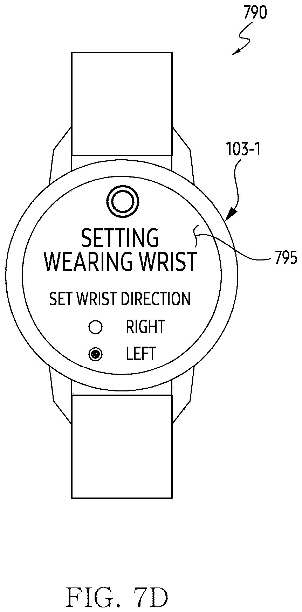

The wearable device 103 according to the embodiments of the present disclosure may provide a notification based on the wearing state and the skin state (or condition). For example, the notification may include visual information (e.g., a visual object of FIGS. 6B, 7B, 7C, and 7D below) guiding a change in the wearing state. In addition, for example, the notification may include auditory information (e.g., sound) or tactile information (e.g., vibration) guiding the change in the wearing state.

The wearable device 103 according to the embodiments of the present disclosure may use a skin type of a user who will wear the wearable device 103, in obtaining a value indicating the degree of damage to the skin based on the information on the skin state (or condition). For example, the skin type may include dry, oily, combination, or sensitive.

In addition, the wearable device 103 according to the embodiments of the present disclosure may perform control for a source of a sensor used for data measurement. For example, the control for the source may include adjust of the amount of light. The sensor may be referred to as an optical sensor. For example, while the wearable device 103 measures optical biometric data, the amount of light of the source may be adjusted. In this case, in the wearing of the wearable device 103 based on the unbalanced pressure, in case that amount of reflected light reaching the light absorbing part of the sensor is small, the wearable device 103 may increase the amount of light emitted from the sensor. As the amount of light increases, damage may occur to the body portion on which the wearable device 103 is worn. In this case, in case that the wound or inflammation based on the unbalanced pressure occurs in the body portion, photosensitive inflammation may be caused. Thus, in case that an unbalance state of wearing pressure is detected while wearing the wearable device 103, the embodiments of the present disclosure may prevent the damage in advance by adjusting the amount of light of the wearable device 103 and guiding the wearing method of the wearable device 103.

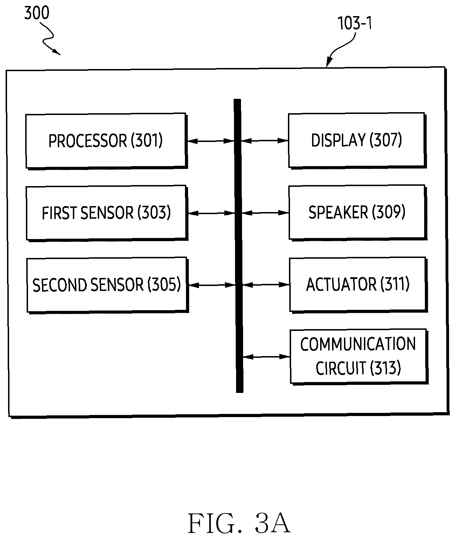

FIG. 3A illustrates an exemplary block diagram of a wearable device worn on a wrist portion of a user.

A wearable device 103-1 of FIG. 3A illustrates an exemplary block diagram 300 of the wearable device 103-1 worn on a wrist portion 203-1 of a user 205 of FIG. 2. For example, the wearable device 103-1 may be referred to as a watch or a smart watch.

Referring to FIG. 3A, the wearable device 103-1 may be connected to an electronic device 101 of FIG. 1 each other based on a wired network and/or a wireless network. For example, the wired network may include a network such as the Internet, a local area network (LAN), a wide area network (WAN), or a combination thereof. For example, the wireless network may include a network such as long term evolution (LTE), 5g new radio (NR), wireless fidelity (WiFi), Zigbee, near field communication (NFC), Bluetooth, Bluetooth low-energy (BLE), or a combination thereof. The wearable device 103-1 may be directly connected to the electronic device 101 or may be indirectly connected through one or more routers and/or access points (APs).

Referring to FIG. 3A, according to an embodiment, the wearable device 103-1 may include at least one of a processor 301, a first sensor 303, a second sensor 305, a display 307, a speaker 309, an actuator 311, or a communication circuit 313. However, the embodiment of the present disclosure is not limited thereto. For example, the processor 301, the first sensor 303, the second sensor 305, the display 307, the speaker 309, the actuator 311, and the communication circuit 313 electronically and/or operably coupled with each other by a communication bus. Hereinafter, an operably coupling of hardware components may mean that a direct connection or an indirect connection between hardware components is established by wire or wirelessly so that the second hardware component is controlled by the first hardware component among the hardware components. Although illustrated based on different blocks, the embodiment is not limited thereto, and a portion (e.g., at least a portion of the processor 301, and the communication circuit 313) of the hardware components illustrated in FIG. 3A may be included in a single integrated circuit, such as a system on a chip (SoC). A type and/or number of the hardware components included in the wearable device 103-1 is not limited as illustrated in FIG. 3A. For example, the wearable device 103-1 may include only a portion of the hardware components illustrated in FIG. 3A.

According to an embodiment, the processor 301 of the wearable device 103-1 may include a hardware component for processing data based on one or more instructions. The hardware component for processing data may include, for example, an arithmetic and logic unit (ALU), a floating point unit (FPU), and a field programmable gate array (FPGA). For example, the hardware component for processing data may include a central processing unit (CPU), a graphics processing unit (GPU), a digital signal processing (DSP), a microcontroller (MCU), and/or a neural processing unit (NPU). The number of the processors 301 may be one or more. For example, the processor 301 may have a structure of a multi-core processor such as a dual core, a quad core, or a hexa core. The processor 301 of FIG. 3A may be applied substantially the same as content of a processor 120 of FIG. 1.

According to an embodiment, the first sensor 303 of the wearable device 103-1 may include at least one sensor for measuring pressure between the wearable device 103-1 and a body portion on which the wearable device 103-1 is worn. For example, the first sensor 303 may be referred to as a wearing pressure sensor, a pressure sensor, and a pressure measurement sensor. For example, the first sensor 303 may include a barometer, a force sensor, and a strain sensor. For example, the barometer may be used to measure the pressure between the wearable device 103-1 and the body portion. For example, the force sensor may include a force sensitive register (FSR) or a force sensitive capacitor (FSC). The force sensor may further include a touch circuit. For example, the strain sensor may detect load deformation according to a pressure applied to the wearable device 103-1.

According to an embodiment, the second sensor 305 of the wearable device 103-1 may include at least one sensor for measuring information (or skin state (or condition)) on a skin of the body portion on which the wearable device 103-1 is worn. For example, the second sensor 305 may be referred to as a skin state (or condition) measurement sensor, an environmental sensor, or a skin measurement sensor. For example, the second sensor 305 may obtain information on the skin based on an electrical method or an optical method. For example, the second sensor 305 using the electrical method may measure the temperature, humidity, and hydration of the skin based on data (e.g., capacitance or impedance) obtained using an electrode (or contact node). For example, the second sensor 305 using the optical method may measure the temperature, humidity, and hydration of the skin based on information in which the emitted light (e.g., near-infrared rays) is absorbed.

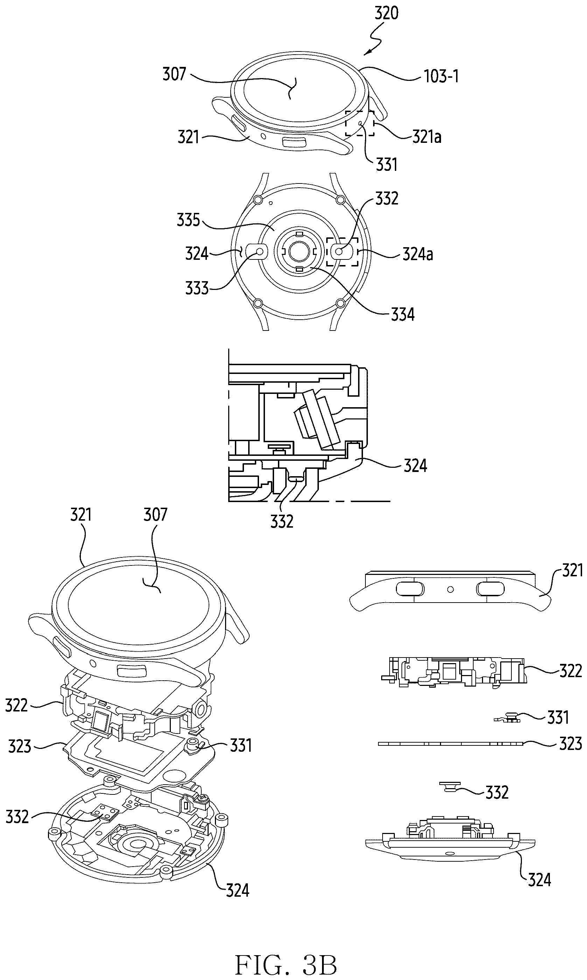

As described above, a specific content of a structure of the wearable device 103-1 including the first sensor 303 and the second sensor 305 will be described in FIGS. 3B to 4B below. In FIG. 3A, an example of the wearable device 103-1 including the first sensor 303 and the second sensor 305 is illustrated, but the embodiment of the present disclosure is not limited thereto. For example, the wearable device 103-1 may further include a barometer to measure the air pressure outside the wearable device 103-1, a heart rate monitor (HRM) to measure pulse rate, an electrocardiogram (ECG), and a bioelectrical impedance analysis (BIA).

According to an embodiment, the display 307 of the wearable device 103-1 may output visualized information to the user. The number of displays 307 included in the wearable device 103 may be one or more. For example, the display 307 may output the visualized information to the user, by being controlled by the processor 301 and/or a graphic processing unit (GPU) (not illustrated). The display 307 may include a flat panel display (FPD), and/or an electronic paper. The FPD may include a liquid crystal display (LCD), a plasma display panel (PDP), a digital mirror device (DMD), one or more light emitting diodes (LEDs), and/or a micro LED. The LED may include an organic LED (OLED). As for a specific content about the display 307 of FIG. 3A, a content about a display module 160 of FIG. 1 may be applied substantially the same.

According to an embodiment, the wearable device 103-1 may include an output means for outputting information in a form other than a form in which information is visualized. For example, the wearable device 103-1 may include the speaker 309 for outputting an acoustic signal or sound. For example, the wearable device 103-1 may include the actuator 311 (or motor) for providing haptic feedback based on vibration.

Although not illustrated in FIG. 3A, according to an embodiment, the wearable device 103-1 may include a module for power supply. For example, the wearable device 103-1 may include a battery. As for a specific content about the battery, a content about a battery 189 of FIG. 1 may be applied substantially the same.

According to an embodiment, the communication circuit 313 of the wearable device 103-1 may include hardware for supporting transmission and/or reception of an electrical signal between the wearable device 103-1 and the electronic device 101 (or a wearable device 103-2). The communication circuit 313 may include, for example, at least one of a modem (MODEM), an antenna, and an optic/electronic (O/E) converter. The communication circuit 313 may support the transmission and/or reception of the electrical signal based on various types of communication means such as ethernet, Bluetooth, Bluetooth low energy (BLE), ZigBee, long term evolution (LTE), and 5G new radio (NR). As for a specific content about the communication circuit 313 of FIG. 3A, a content about a communication module 190 and/or an antenna module 197 of FIG. 1 may be applied substantially the same.

Although not illustrated in FIG. 3A, the wearable device 103-1 may include memory. The memory may include a hardware component for storing data and/or an instruction inputted to the processor 301 and/or outputted from the processor 301. The memory may include, for example, volatile memory such as random-access memory (RAM) and/or non-volatile memory such as readonly memory (ROM). The volatile memory may include, for example, at least one of dynamic RAM (DRAM), static RAM (SRAM), Cache RAM, and pseudo SRAM (PSRAM). The non-volatile memory may include, for example, at least one of programmable ROM (PROM), erasable PROM (EPROM), electrically erasable PROM (EEPROM), flash memory, hard disk, compact disk, and embedded multi media card (eMMC). As for a specific content about the memory of FIG. 3A, a content about memory 130 of FIG. 1 may be applied substantially the same.

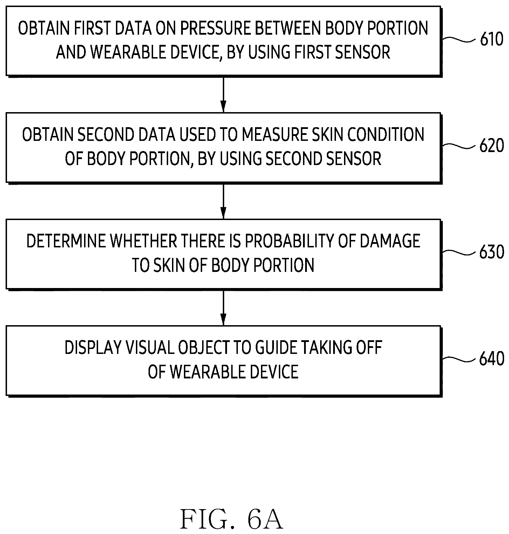

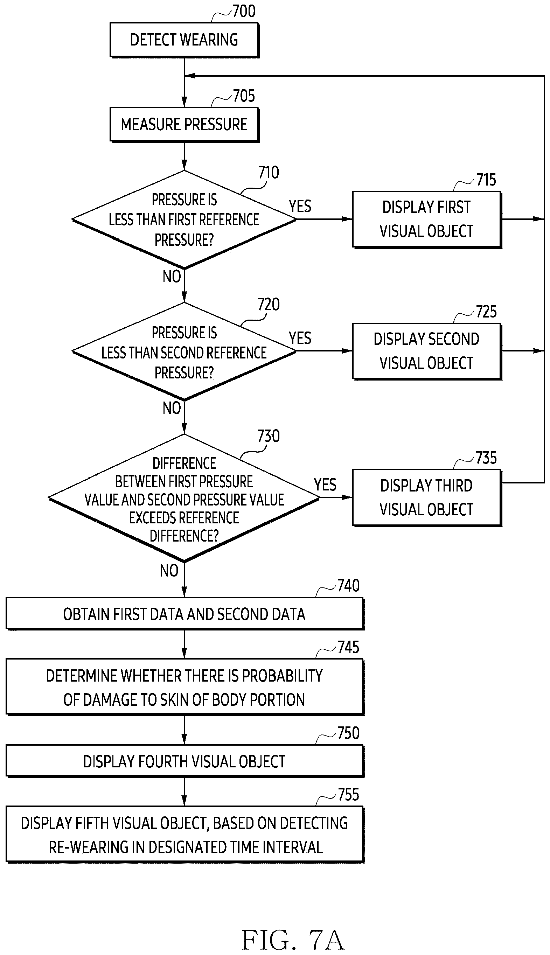

Referring to FIG. 3A, according to an embodiment, in the memory of the wearable device 103-1, one or more instructions (or commands) indicating a calculation and/or operation to be performed by the processor 301 of the wearable device 103-1 on data may be stored. A set of the one or more instructions may be referred to as a program, a firmware, an operating system, a process, a routine, a sub-routine, and/or an application. Hereinafter, the fact that the application is installed in an electronic device (e.g., the wearable device 103-1) means that the one or more instructions provided in the form of the application are stored in the memory, and the one or more applications are stored in an executable format (e.g., a file with an extension designated by the operating system of the wearable device 103-1) by the processor of the electronic device. According to an embodiment, the wearable device 103-1 may perform an operation of FIGS. 6A and 7A, by executing the one or more instructions stored in the memory. For example, the one or more instructions, when executed by the processor 301, may cause the wearable device 103-1 to perform at least some of the operations of FIG. 6A, or at least some of the operations of FIG. 7A.

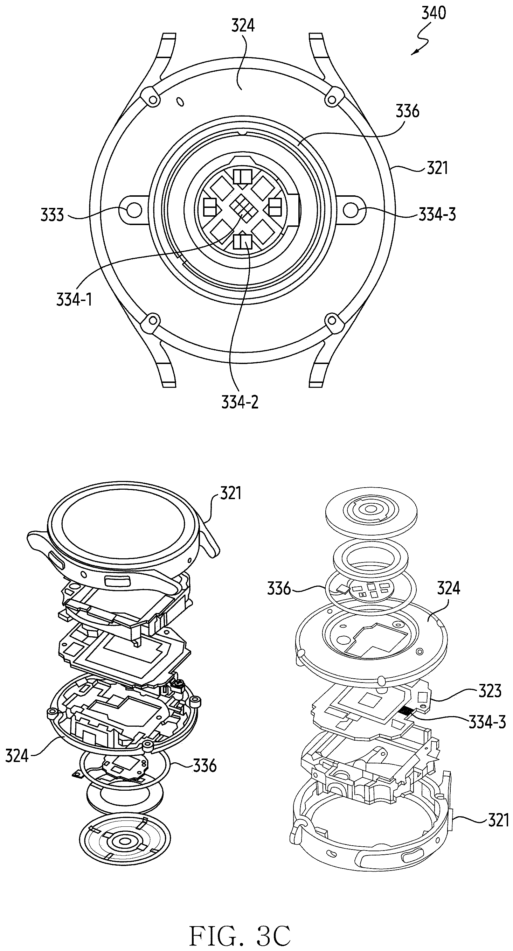

FIGS. 3B and 3C illustrate examples of components included in a wearable device worn on a wrist portion of a user.

FIGS. 3B and 3C illustrate examples of components included in the wearable device 103-1 of FIG. 3A. FIG. 3B illustrates an example 320 of the wearable device 103-1 including a first sensor 303 including a barometer and a second sensor 305 using an electrical method. FIG. 3C illustrates an example 340 of the wearable device 103-1 including the first sensor 303 including a force sensor and the second sensor 305 using an optical method.

Referring to the example 320 of FIG. 3B, the wearable device 103-1 may include a display 307, a housing 321, a bracket 322, a printed circuit board (PCB) 323, and a plate 324. In an embodiment, the housing 321 may form an exterior of the wearable device 103-1. For example, the housing 321 may include the display 307 positioned on a front plate, the plate 324 (or rear plate), and a frame between the front plate and the plate 324. In an embodiment, the bracket 322 may be disposed inside the housing 321. For example, the bracket 322 may be disposed inside the frame. The bracket 322 may be positioned between the display 307 and the PCB 323. For example, the display 307 may be disposed on one surface of the bracket 322, and the PCB 323 may be disposed on the other surface thereof. The bracket 322 may support the display 307 and the PCB 323. The bracket 322 may be formed of a metal material and/or a non-metal material (e.g., polymer). In an embodiment, the PCB 323 may be equipped with a processor (e.g., a processor 301 of FIG. 3A), memory, and/or at least some (e.g., a sensor of FIG. 3A) of the components. The processor may include, for example, one or more of a central processing unit, an application processor, a graphic processing unit (GPU), an application processor, sensor processor, or a communication processor. The memory may include, for example, volatile memory or non-volatile memory. The interface may include, for example, a high definition multimedia interface (HDMI), a universal serial bus (USB) interface, an SD card interface, and/or an audio interface. According to an embodiment, the plate 324 may be connected to the PCB 323. For example, the plate 324 may include an area in contact with the body portion of the user of the wearable device 103-1.

Referring to the example 320, the wearable device 103-1 may include a first barometer 331. For example, the wearable device 103-1 may include the first barometer 331 that measures external air pressure through an area 321a of the housing 321. For example, the first barometer 331 may be disposed in the area 321a in which a hole for measuring the external air pressure is not covered by the body portion. For example, the first barometer 331 may be used to measure the air pressure outside the wearable device 103-1. For example, the first barometer 331 may be positioned in the area 321a of a side frame of the housing 321. For example, the first barometer 331 may be disposed on the PCB 323.

Referring to the example 320, the wearable device 103-1 may include a second barometer 332. The second barometer 332 may be included in the first sensor 303 of FIG. 3A. For example, the wearable device 103-1 may include the second barometer 332 that measures air pressure between the wearable device 103-1 and the body portion (e.g., the wrist portion). For example, the second barometer 332 may be disposed in an area 324a in which the hole is covered by the body portion. For example, the second barometer 332 may be used to measure the air pressure between the wearable device 103-1 and the body portion of the wearable device 103-1. The air pressure between the wearable device 103-1 and the body portion may be referred to as wearing pressure of the wearable device 103-1. In other words, the air pressure between the wearable device 103-1 and the body portion may be referred to as pressure felt by the user according to the wearing of the wearable device 103-1. For example, the second barometer 332 may be positioned in the area 324a of the plate 324. For example, the second barometer 332 may be disposed on the PCB 323. The second barometer 332 may be implemented as the same type of barometer as the first barometer 331, or may be implemented as a different barometer.

According to an embodiment, the wearable device 103-1 may measure the pressure on the body portion, based on a difference between a first pressure value measured by the first barometer 331 and a second pressure value measured by the second barometer 332. For example, the pressure on the body portion may be referred to as wearing pressure, measurement pressure, or contact pressure. For example, the wearable device 103-1 may recognize that the smaller the difference, the lower the pressure on the body portion. For example, the wearable device 103-1 may distinguish a level of the pressure on the body portion, based on the difference and the reference pressures. For example, the level may include contact, low, medium, and high. The contact of the level may indicate pressure at a level at which the wearable device 103-1 and the body portion start to contact.

Referring to the example 320, the wearable device 103-1 may include a temperature sensor 333 for measuring the temperature of the body portion and a humidity sensor 334 for measuring a humidity (or hydration) of the body portion. For example, the temperature sensor 333 and the humidity sensor 334 may be included in the second sensor 305 of FIG. 3A. For example, the temperature sensor 333 and the humidity sensor 334 may obtain data for measuring the temperature or the humidity through the plate 324. For example, the temperature sensor 333 and the humidity sensor 334 may be disposed inside the plate 324 or the housing 321 adjacent to the plate 324. In FIG. 3B, an example of the temperature sensor 333 using the electrical method is illustrated, but an embodiment of the present disclosure is not limited thereto. For example, the wearable device 103-1 may include the temperature sensor 333 using the optical method. In this case, the temperature sensor 333 may be disposed at a position close to the source to reduce damage to the body portion.

Referring to the example 320, the wearable device 103-1 may further include an additional sensor 335 such as an electrocardiogram (ECG) and a bioelectrical impedance analysis (BIA). For example, the sensor 335 may be disposed inside the plate 324 or the housing 321 adjacent to the plate 324.

Referring to the example 340 of FIG. 3C, the wearable device 103-1 may be substantially the same as the wearable device 103-1 of the example 320 of FIG. 3B. In other words, the wearable device 103-1 of the example 340 may include the display 307, the housing 321, the bracket 322, the printed circuit board (PCB) 323, and the plate 324. In order to avoid overlapping descriptions, the same contents as those in FIG. 3B will be omitted below.

Referring to the example 340, the wearable device 103-1 may include the temperature sensor 333 for measuring the temperature of the skin of the body portion, a sensor 334-1 for measuring oxygen information on the skin, a sensor 334-2 for measuring sweat of the body portion, and a humidity sensor 334-3 for measuring humidity of the body portion. The temperature sensor 333, the sensor 334-1, the sensor 334-2, and the humidity sensor 334-3 may be included in the second sensor 305 of FIG. 3A. For example, the temperature sensor 333, the sensor 334-1, the sensor 334-2, and the humidity sensor 334-3 may obtain data for measuring the temperature or the humidity through the plate 324. For example, the humidity sensor 334-3 may be disposed on the PCB 323.

Referring to the example 340, the wearable device 103-1 may include a force sensor 336. The force sensor 336 may be included in the first sensor 303 of FIG. 3A. For example, the wearable device 103-1 may include the force sensor 336 that measures pressure between the wearable device 103-1 and the body portion. For example, the pressure may indicate degree to which the body portion is pressed by the wearable device 103-1. For example, in case of tightening a strap of the wearable device 103-1, the pressure may increase. For example, the force sensor 336 may obtain data for measuring the pressure through the plate 324. For example, the force sensor 336 may be disposed in the housing 321.

Although not illustrated in the example 340, the wearable device 103-1 may further include an additional sensor such as the electrocardiogram (ECG) and the bioelectrical impedance analysis (BIA).

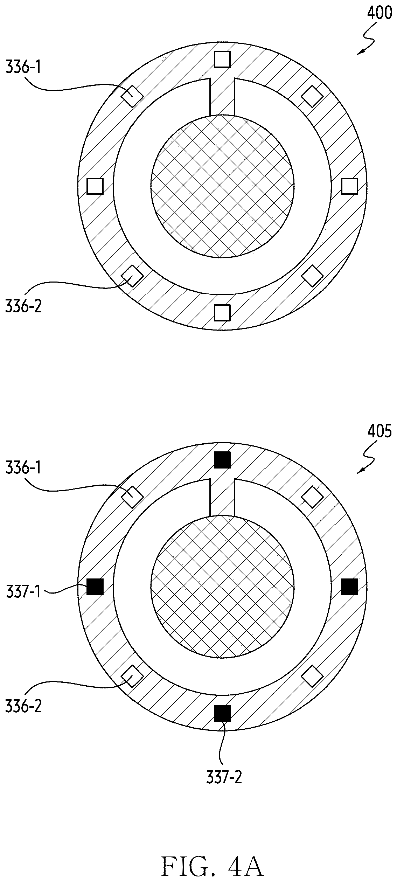

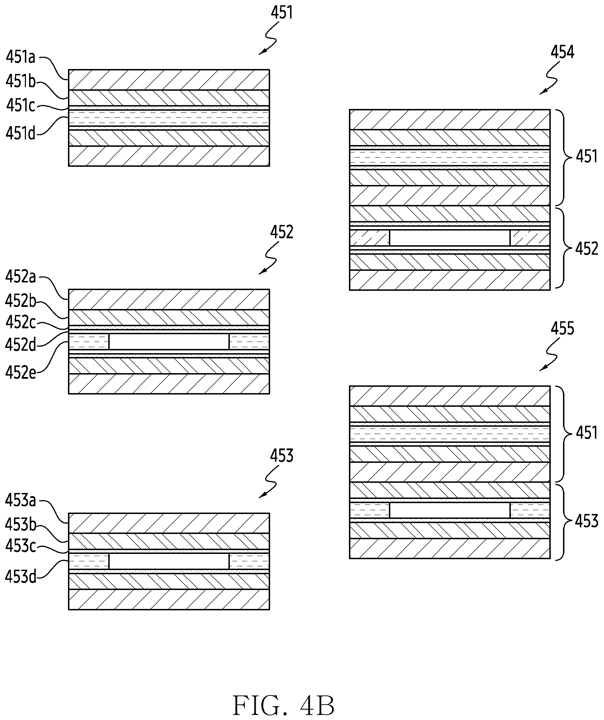

FIGS. 4A and 4B illustrate examples of a first sensor included in a wearable device.

A wearable device 103-1 of FIGS. 4A and 4B may indicate an example of the wearable device 103-1 of FIG. 3A. For example, the force sensors 336-1, 336-2, 337-1, and 337-2 of the wearable device 103-1 may be included in a first sensor 303 of the wearable device 103-1. For example, the force sensors 336-1, 336-2, 337-1, and 337-2 may indicate an example of a force sensor 336 of FIG. 3C.

Referring to FIG. 4A, examples 400 and 405 of the wearable device 103-1 including a plurality of force sensors are illustrated. Referring to the example 400, the wearable device 103-1 may include the force sensors 336-1 and 336-2 of the same type. For example, the wearable device 103-1 may include eight force sensors. Referring to the example 405, the wearable device 103-1 may include a plurality of types of force sensors 336-1, 336-2, 337-1, and 337-2. For example, the force sensors 336-1 and 336-2 may indicate a first type of force sensor. Alternatively, the force sensors 337-1 and 337-2 may indicate a second type of force sensor different from the first type. For example, the wearable device 103-1 may include four force sensors of the first type and four force sensors of the second type. For example, the wearable device 103-1 may include seven or less force sensors or nine or more force sensors. As illustrated in the example 340 of FIG. 3C, the plurality of force sensors may measure the pressure between the wearable device 103-1 and the body portion, through the plate 324 of the wearable device 103-1. However, the embodiment of the present disclosure is not limited thereto. In addition, for example, the wearable device 103-1 may include force sensors that are disposed at a different position from force sensors at a position illustrated in the examples 400 and 405.

Referring to FIG. 4B, examples 451, 452, 453, 454, and 455 of various types of the plurality of force sensors included in the wearable device 103-1 of FIG. 4A are illustrated. For example, the various types may include the first type and the second type. For example, the first type may be an FSC. For example, the second type may be an FSR.

Referring to the example 451, the force sensor may be implemented as the FSC. For example, the FSC may include a plurality of layers. For example, the plurality of layers may include a substrate layer 451a, an adhesive layer 451b, a conductive layer 451c, and a compressible layer 451d. Referring to the example 451, a plurality of substrate layers 451a, a plurality of adhesive layers 451b, a plurality of conductive layers 451c, and the compressible layer 451d may be included. The force sensor may be used together with a touch IC. The FSC may be referred to as a capacitive method of force sensor.

Referring to the example 452, the force sensor may be implemented as the FSR. For example, the FSR may include a plurality of layers. For example, the plurality of layers may include a substrate layer 452a, an adhesive layer 452b, a conductive layer 452c, a pressure sensitive layer 452d, and a compressible layer 452e. Referring to the example 452, a plurality of substrate layers 452a, a plurality of adhesive layers 452b, a plurality of conductive layers 452c, a plurality of pressure sensitive layers 452d, and the compressible layer 452e may be included. At least a portion of the compressible layer 452e may include an air layer. The FSR may be referred to as a resistance method of force sensor. The FSR of the example 452 may be referred to as a thru mode FSR.

Referring to the example 453, the force sensor may be implemented as the FSR. For example, the FSR may include a plurality of layers. For example, the plurality of layers may include a substrate layer 453a, an adhesive layer 453b, a conductive layer 453c, and a compressible layer 453d. Referring to the example 453, a plurality of substrate layers 453a, a plurality of adhesive layers 453b, a plurality of conductive layers 453c, and the compressible layer 453d may be included. At least a portion of the compressible layer 453e may include an air layer. The FSR may be referred to as the resistance method of force sensor. The FSR of the example 453 may be referred to as a shunt mode FSR.

Referring to the example 454 and the example 455, the force sensor may be implemented based on a combination of the FSC and the FSR. Referring to the example 454, the force sensor may include the FSC of the example 451 and the FSR of the example 452. Referring to the example 455, the force sensor may include the FSC of the example 451 and the FSR of the example 453. For example, the FSR and the FSR may be stacked.

According to an embodiment, although not illustrated in FIG. 4B, the force sensor may be implemented as a combination of the force sensors of the examples 451, 452, 453, 454, and 455. In addition, for example, the first sensor for measuring the pressure may use a strain sensor or a barometer as well as the force sensor.

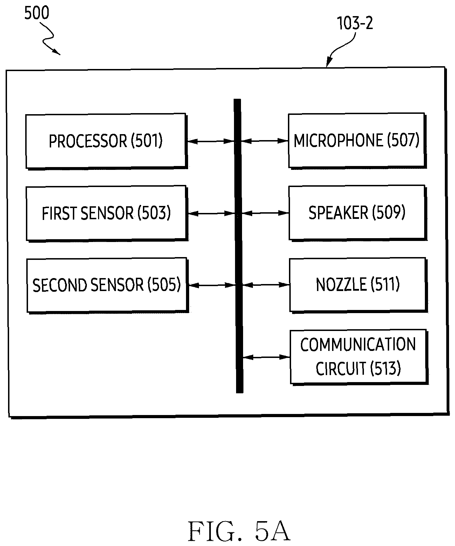

FIG. 5A illustrates an exemplary block diagram of a wearable device worn on an ear portion of a user.

A wearable device 103-2 of FIG. 5A illustrates an exemplary block diagram 500 of the wearable device 103-2 worn on an ear portion 203-2 of a user 205 of FIG. 2. For example, the wearable device 103-2 may be referred to as earbuds, earphone, or true wireless stereo (TWS).

Referring to FIG. 5A, the wearable device 103-2 may be connected to the electronic device 101 of FIG. 1 each other based on a wired network and/or a wireless network. For example, the wired network may include a network such as the Internet, a local area network (LAN), a wide area network (WAN), or a combination thereof. For example, the wireless network may include a network such as long term evolution (LTE), 5g new radio (NR), wireless fidelity (WiFi), Zigbee, near field communication (NFC), Bluetooth, Bluetooth low-energy (BLE), or a combination thereof. The wearable device 103-2 may be directly connected to the electronic device 101 or may be indirectly connected through one or more routers and/or access points (APs).

Referring to FIG. 5A, according to an embodiment, the wearable device 103-2 may include at least one of a processor 501, a first sensor 503, a second sensor 505, a microphone 507, a speaker 509, a nozzle 511, or a communication circuit 513. However, the embodiment of the present disclosure is not limited thereto. For example, the processor 501, the first sensor 503, the second sensor 505, the microphone 507, the speaker 509, the nozzle 511, and the communication circuit 513 electronically and/or operably coupled with each other by a communication bus. Hereinafter, an operably coupling of hardware components may mean that a direct connection or an indirect connection between hardware components is established by wire or wirelessly so that the second hardware component is controlled by the first hardware component among the hardware components. Although illustrated based on different blocks, the embodiment is not limited thereto, and a portion (e.g., at least a portion of the processor 501, and the communication circuit 513) of the hardware components illustrated in FIG. 5A may be included in a single integrated circuit, such as a system on a chip (SoC). A type and/or number of the hardware components included in the wearable device 103-2 is not limited as illustrated in FIG. 5A. For example, the wearable device 103-2 may include only a portion of the hardware components illustrated in FIG. 5A.

According to an embodiment, the processor 501 of the wearable device 103-2 may include a hardware component for processing data based on one or more instructions. The hardware component for processing data may include, for example, an arithmetic and logic unit (ALU), a floating point unit (FPU), and a field programmable gate array (FPGA). For example, the hardware component for processing data may include a central processing unit (CPU), a graphics processing unit (GPU), a digital signal processing (DSP), a microcontroller (MCU), and/or a neural processing unit (NPU). The number of the processors 501 may be one or more. For example, the processor 501 may have a structure of a multi-core processor such as a dual core, a quad core, or a hexa core. The processor 501 of FIG. 5A may be applied substantially the same as content of a processor 120 of FIG. 1.

According to an embodiment, the first sensor 503 of the wearable device 103-2 may include at least one sensor for measuring movement of the wearable device 103-2. For example, the first sensor 503 may be referred to as a movement sensor, a movement measurement sensor, or a proximity sensor. For example, the first sensor 503 may include an accelerometer. For example, the accelerometer may be used to detect whether to wear (or whether to contact) between the wearable device 103-2 and the body portion. For example, the wearable device 103-2 may recognize the movement of the wearable device 103-2 and may detect the contact between the wearable device 103-2 and the body portion according to amount of change in stimulus, based on the accelerometer.

According to an embodiment, the second sensor 505 of the wearable device 103-2 may include at least one sensor for measuring information (or skin state (or condition)) on a skin of the body portion on which the wearable device 103-2 is worn. For example, the second sensor 505 may be referred to as a skin state (or condition) measurement sensor, an environmental sensor, or a skin measurement sensor. For example, the second sensor 505 may obtain information on the skin based on an electrical method or an optical method. For example, the second sensor 505 using the electrical method may measure the temperature, humidity, and hydration of the skin by using an electrode (or contact node). For example, the second sensor 505 using the optical method may measure the temperature, humidity, and hydration of the skin based on information in which the emitted light (e.g., near-infrared rays) is absorbed.

In FIG. 5A, an example of the wearable device 103-2 including the first sensor 503 and the second sensor 505 is illustrated, but the embodiment of the present disclosure is not limited thereto. For example, the wearable device 103-2 may further include a barometer to measure the air pressure outside the wearable device 103-2, a heart rate monitor (HRM) to measure pulse rate, an electrocardiogram (ECG), and a bioelectrical impedance analysis (BIA).

According to an embodiment, the wearable device 103-2 may include a g 507 for obtaining sound (e.g., acoustic signal) inputted from the outside of the wearable device 103-2. For example, the microphone 507 may include a plurality of microphones. The microphone 507 may include an internal microphone and an external microphone identified based on a direction in which the acoustic signal is obtained. For example, the internal microphone may include at least one microphone for obtaining the acoustic signal from a first direction toward the body portion, in a state that the wearable device 103-2 is worn on the body portion. For example, the external microphone may include at least one microphone for obtaining the acoustic signal from a second direction different from the first direction, in the state that the wearable device 103-2 is worn on the body portion. For example, the external microphone may include a main mic and a sub mic for obtaining the acoustic signal from the second direction. For example, the main mic may be used to obtain the acoustic signal from the second direction. For example, the sub mic may be used to obtain the acoustic signal auxiliary to the main mic, in case that the main mic is not used, or in case that the quality of the acoustic signal obtained from the main mic is less than or equal to a designated quality. For example, the microphone 507 may be an electronic condenser microphone (ECM) or a micro electro mechanical system (MEMS), but is not limited thereto. As for a specific content about the microphone 507 of FIG. 5A, a content about an input module 150 of FIG. 1 may be applied substantially the same.

According to an embodiment, the wearable device 103-2 may include the nozzle 511. For example, the nozzle 511 may be referred to as an acoustic port. For example, the nozzle 511 may be a path through which the wearable device 103-2 is supported in the body portion and the sound outputted from the wearable device 103-2 passes, when the wearable device 103-2 is worn on the body portion. For example, the nozzle 511 may be connected to an eartip implemented with a conductive member. At least a portion of the eartip implemented with the conductive member may be used as the second sensor 505 for detecting the hydration of the skin of the body portion. Alternatively, for example, at least a portion of the nozzle 511 may be used as the second sensor 505 for detecting the hydration of the skin of the body portion.

According to an embodiment, the wearable device 103-2 may include an output means for outputting information in a form other than a form in which information is visualized. For example, the wearable device 103-2 may include the speaker 509 for outputting the acoustic signal or the sound. However, the embodiment of the present disclosure is not limited thereto. For example, the wearable device 103-2 may include an actuator (or motor) for providing haptic feedback based on vibration.

Although not illustrated in FIG. 5A, according to an embodiment, the wearable device 103-2 may include a module for power supply. For example, the wearable device 103-2 may include a battery. As for a specific content about the battery, a content about a battery 189 of FIG. 1 may be applied substantially the same.

According to an embodiment, it is possible to recognize whether the wearable device 103-2 is in a normal wearing state for the body portion. The normal wearing state may indicate a state in which the wearable device 103-2 is in contact with the body portion and the wearable device 103-2 is supported so as not to be separated from the body portion without external force. For example, the normal wearing state may be recognized based on the reflection of sound (e.g., chirp or click) for the entire frequency outputted through the speaker 509, a sound difference between the internal microphone and the external microphone of the microphone 507, or a level of physical shielding of the sound played by the speaker 509. For example, the wearable device 103-2 may output sound in the entire frequency band, such as the chirp or the click, through the speaker 509, and may obtain a response curve of the sound reflected through an eardrum in the body portion through the internal microphone. Accordingly, the wearable device 103-2 may recognize the normal wearing state by checking a frequency response curve corresponding to a shape and volume level of an ear canal of the body portion through analysis of each frequency of the obtained sound. Alternatively, for example, the wearable device 103-2 may recognize the normal wearing state, by outputting the sound through the speaker 509 and comparing a magnitude of the sound obtained through each of the internal and external microphones to determine whether the physical shielding level is satisfied. Alternatively, for example, the wearable device 103-2 may recognize the normal wearing state, by checking the contact level between the nozzle 511 and the ear canal, by measuring impedance through the nozzle 511 implemented with the conductive member (or a conductive eartip connected to the nozzle 511).

According to an embodiment, the communication circuit 513 of the wearable device 103-2 may include hardware for supporting transmission and/or reception of an electrical signal between the wearable device 103-2 and the electronic device 101. The communication circuit 513 may include, for example, at least one of a modem (MODEM), an antenna, and an optic/electronic (O/E) converter. The communication circuit 513 may support the transmission and/or reception of the electrical signal based on various types of communication means such as ethernet, Bluetooth, Bluetooth low energy (BLE), ZigBee, long term evolution (LTE), and 5G new radio (NR). As for a specific content about the communication circuit 513 of FIG. 5A, a communication module 190 and/or an antenna module 197 of FIG. 1 may be applied substantially the same.

Although not illustrated in FIG. 5A, the wearable device 103-2 may include memory. The memory may include a hardware component for storing data and/or an instruction inputted to the processor 501 and/or outputted from the processor 501. The memory may include, for example, volatile memory such as random-access memory (RAM) and/or non-volatile memory such as readonly memory (ROM). The volatile memory may include, for example, at least one of dynamic RAM (DRAM), static RAM (SRAM), Cache RAM, and pseudo SRAM (PSRAM). The non-volatile memory may include, for example, at least one of programmable ROM (PROM), erasable PROM (EPROM), electrically erasable PROM (EEPROM), flash memory, hard disk, compact disk, and embedded multi media card (eMMC). As for a specific content about the memory of FIG. 5A, a content about memory 130 of FIG. 1 may be applied substantially the same.

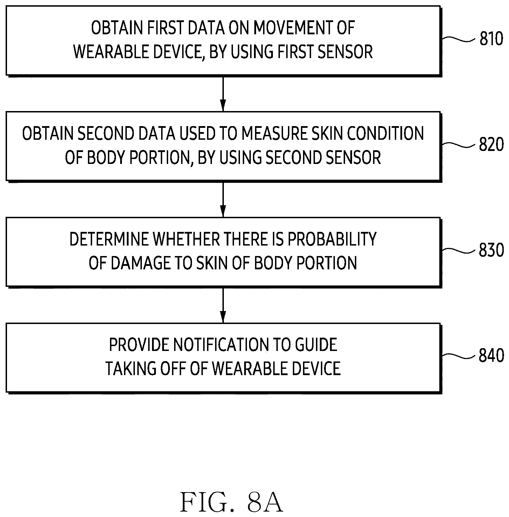

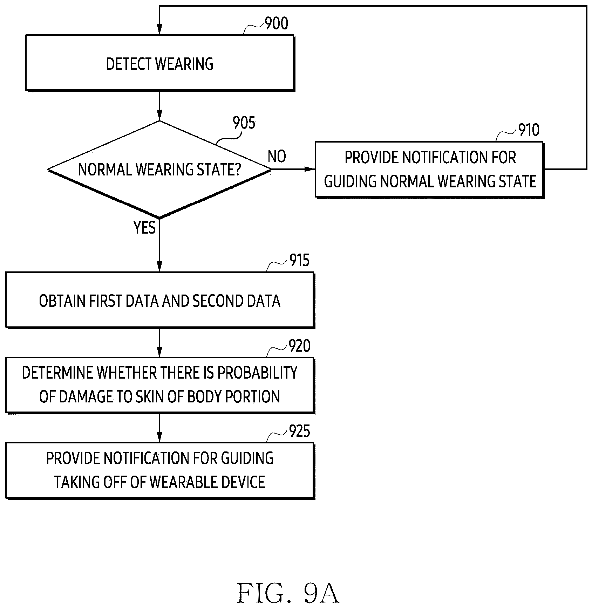

Referring to FIG. 5A, according to an embodiment, in the memory of the wearable device 103-2, one or more instructions (or commands) indicating a calculation and/or operation to be performed by the processor 501 of the wearable device 103-2 on data may be stored. A set of the one or more instructions may be referred to as a program, a firmware, an operating system, a process, a routine, a sub-routine, and/or an application. Hereinafter, the fact that the application is installed in an electronic device (e.g., the wearable device 103-2) means that the one or more instructions provided in the form of the application are stored in the memory, and the one or more applications are stored in an executable format (e.g., a file with an extension designated by the operating system of the wearable device 103-2) by the processor of the electronic device. According to an embodiment, the wearable device 103-2 may perform an operation of FIGS. 8A and 9A, by executing the one or more instructions stored in the memory. For example, the one or more instructions, when executed by the processor 501, may cause the wearable device 103-2 to perform at least some of the operations of FIG. 8A, or at least some of the operations of FIG. 9A.

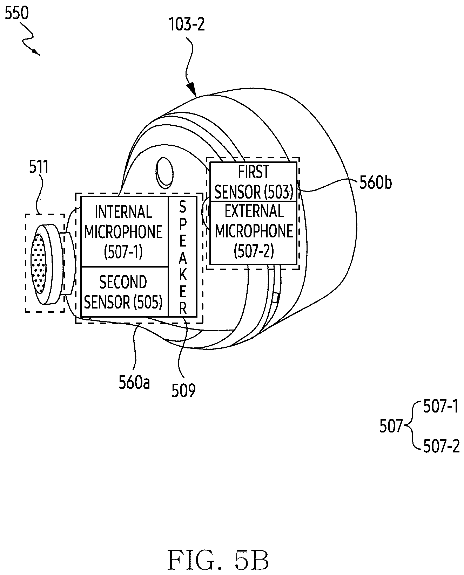

FIG. 5B illustrates an example of components included in a wearable device worn on an ear portion of a user. FIG. 5B illustrates an example 550 of components included in a wearable device 103-2 of FIG. 5A.

Referring to the example 550 of FIG. 5B, the wearable device 103-2 may include a first sensor 503, a second sensor 505, a microphone 507, a speaker 509, and a nozzle 511. A shape and the components of the wearable device 103-2 illustrated in the example 550 of FIG. 5B are merely exemplary, and the embodiment of the present disclosure is not limited thereto.

For example, the wearable device 103-2 may include a nozzle 511 that is inserted into an ear canal of a body portion (e.g., the ear portion) of the user where the wearable device 103-2 is worn. For example, the nozzle 511 may be supported in a state of being inserted into the ear canal. For example, the nozzle 511 may be a portion of a path of sound outputted through the speaker 509 of the wearable device 103-2.

For example, the wearable device 103-2 may include the second sensor 505 for obtaining a temperature, a humidity (or hydration), or oxygen information of the body portion. The second sensor 505 may be disposed in an area 560a adjacent to the nozzle 511 in a housing of the wearable device 103-2. In addition, for example, the wearable device 103-2 may include an internal microphone 507-1 in the area 560a.

For example, the wearable device 103-2 may include the first sensor 503. For example, the wearable device 103-2 may include the first sensor 503 for obtaining data on movement used to recognize whether the wearable device 103-2 and the body portion are in contact with each other, in the area 560b. For example, the wearable device 103-2 may include an external microphone 507-2, in the area 560b.

FIG. 6A illustrates an example of an operation flow of a method of recognizing the degree of damage to a body portion on which a wearable device is worn and displaying a visual object guiding a wearing method. FIG. 6B illustrates an example of a visual object guiding a wearing method of a wearable device.

At least a portion of the method of FIG. 6A may be performed by a wearable device 103-1 of FIG. 3A. For example, at least a portion of the method may be controlled by a processor 301 of the wearable device 103-1. In the following embodiment, each operation may be performed sequentially, but is not necessarily performed sequentially. For example, an order of each operation may be changed, and at least two operations may be performed in parallel.

In operation 610, the wearable device 103-1 may obtain first data on pressure between the body portion and the wearable device 103-1, by using a first sensor. For example, the wearable device 103-1 may obtain the first data on the pressure, by using the first sensor, based on recognizing that the wearable device 103-1 is worn on the body portion. For example, the body portion may include a wrist portion of the user. However, the embodiment of the present disclosure is not limited thereto. For example, it may include a finger portion of the user.

For example, the first sensor may include a first sensor 303 of FIG. 3A. For example, the first sensor may include at least one of a barometer, a force sensor, or a strain sensor. For example, the first data may be referred to as a sensor value obtained through the first sensor.

For example, the wearable device 103-1 may recognize that the wearable device 103-1 is worn on the body portion, based on at least one sensor included in the wearable device 103-1. For example, the wearable device 103-1 may recognize the wearing, based on amount of change in recognized pressure, based on the first sensor. Alternatively, for example, the wearable device 103-1 may recognize the wearing, based on a second sensor for obtaining information on the skin of the body portion. Alternatively, for example, the wearable device 103-1 may recognize the wearing, based on another sensor different from the first sensor and the second sensor. The wearing may include the wearable device 103-1 starting to contact the body portion.

In operation 620, the wearable device 103-1 may obtain second data used to measure a skin state (or condition) of the body portion, by using the second sensor. For example, the wearable device 103-1 may obtain the second data used to measure the skin state (or condition) of the body portion, by using the second sensor, while the wearable device 103-1 is worn on the body portion. For example, the skin state (or condition) may include at least one of a temperature, a humidity (or hydration), or active oxygen of the body portion.

For example, the second sensor may include a second sensor 305 of FIG. 3A. For example, the second sensor may include at least one of a sensor for detecting sweat of the body portion, a sensor for detecting the humidity of the body portion, a sensor for detecting the temperature of the body portion, or a sensor for detecting the active oxygen (or antioxidant degree) of the body portion. For example, the second data may be referred to as a sensor value obtained through the second sensor.

In operation 630, the wearable device 103-1 may determine (or predict) whether there is a probability of damage to the skin of the body portion. For example, the wearable device 103-1 may determine whether there is the probability of damage to the skin, based on a length of time worn on the body portion, the first data, and the second data. For example, the probability of damage may be referred to as degree of damage to the skin, degree of damage prediction of the skin, or state of damage to the skin.

For example, the wearable device 103-1 may obtain a value indicating the degree of damage to the skin, based on the length of time when the wearable device 103-1 is worn on the body portion, the first data, and the second data. For example, the length of the time may indicate a time interval from a timing at which the wearable device 103-1 starts to recognize the wearing on the body portion.

According to an embodiment, the value may be identified based on at least two or more parameters of the temperature, the humidity, the active oxygen, the antioxidant degree, the length of the time, or the pressure. For example, the value may be proportional to the temperature. For example, the value may be proportional to the humidity. For example, the value may be proportional to the active oxygen. For example, the value may be inversely proportional to the antioxidant degree. The value may be proportional to the length of the time. For example, the value may be proportional to a magnitude of the value obtained by subtracting the appropriate pressure from the pressure. For example, the appropriate pressure may indicate a pressure value between the body portion and the wearable device 103-1, when the wearable device 103-1 is in the normal wearing state for the body portion. The normal wearing state may indicate a state in which the wearable device 103-1 is in contact with the body portion and the wearable device 103-1 is supported so as not to be separated from the body portion without external force. For example, as the value increases, the barrier of the skin may be further deteriorated. The value may be referred to as a deterioration level of skin barrier.

According to an embodiment, the value may be identified further based on a skin type. For example, the skin type may include dry, oily, combination, or sensitive of the skin. For example, the information on the skin type may be obtained based on an input of the user. Alternatively, for example, the skin type may be identified by using a statistical model based on a designated number of the second data. For example, the statistical model may use machine learning. For example, the machine learning may be learned based on the designated number of the second data.

In operation 640, the wearable device 103-1 may display a visual object to guide taking off (or taking off wearing) of the wearable device 103-1. For example, based on determining that the probability of damage is happened, the wearable device 103-1 may display the visual object to guide the taking off of the wearable device 103-1 on the body portion through the display 307.

According to an embodiment, the wearable device 103-1 may compare the reference value and the value. For example, the wearable device 103-1 may recognize whether the value exceeds the reference value. For example, the reference value may be a value for indicating that the barrier of the skin is deteriorated by a designated level. For example, in case that the value exceeds the reference value, the wearable device 103-1 may determine that there is the probability of damage (or that the probability of damage may be happened).

According to an embodiment, the reference value may be changed according to parameters used to identify the value. For example, in case that parameters used to identify the value are the temperature and the humidity, the reference value may be a first reference value. Alternatively, for example, in case that the parameters used to identify the value are the temperature, the humidity, and the pressure, the reference value may be a second reference value different from the first reference value. Alternatively, for example, in case that the parameters used to identify the values are the active oxygen and the wearing time, the reference value may be a third reference value different from the first reference value and the second reference value.

According to an embodiment, the wearable device 103-1 may control the amount of light used to obtain data through the first sensor or the second sensor while displaying the visual object. For example, the wearable device 103-1 may reduce the amount of light based on the value exceeding the reference value. For example, the amount of light may indicate the amount of light emitted to obtain data through the first sensor or the second sensor.

According to an embodiment, the visual object may include visual information for guiding the taking off of the wearable device 103-1. For a specific example related to this, refer to FIG. 6B below.

FIG. 6B illustrates an example 650 displayed by visual information 660 for guiding the release. For example, in case that the value exceeds the reference value, the wearable device 103-1 may display the visual information 660 on the display 307. For example, the visual information 660 may include an image and text indicating that rest is necessary. For example, the text may include, "For the health of your wrist, take off your watch to clean and remove moisture. Take off your watch briefly or wear it on the opposite hand.". The visual information 660 may include information for guiding the wearable device 103-1 to be worn on a wrist different from the wrist being worn. The example 650 illustrated in FIG. 6B is only for convenience of description, and the embodiment of the present disclosure are not limited thereto.

FIG. 7A illustrates an example of an operation flow of a method in which a wearable device worn on a wrist portion of a user guides a wearing method. FIGS. 7B to 7D illustrate examples of a visual object guiding a wearing method of a wearable device.

At least a portion of the method of FIG. 7A may be performed by a wearable device 103-1 of FIG. 3A. For example, at least a portion of the method may be controlled by a processor 301 of the wearable device 103-1. In the following embodiment, each operation may be performed sequentially, but is not necessarily performed sequentially. For example, an order of each operation may be changed, and at least two operations may be performed in parallel.

In operation 700, the wearable device 103-1 may detect wearing. For example, the wearable device 103-1 may detect the wearing on a body portion including the wrist portion. For example, the wearable device 103-1 may recognize that the wearable device 103-1 is worn on the body portion, based on at least one sensor included in the wearable device 103-1. For example, the wearable device 103-1 may recognize the wearing, based on amount of change in recognized pressure, based on a first sensor (e.g., a first sensor 303 of FIG. 3A). Alternatively, for example, the wearable device 103-1 may recognize the wearing, based on a second sensor (e.g., a second sensor 305 of FIG. 3A) for obtaining information on the skin of the body portion. Alternatively, for example, the wearable device 103-1 may recognize the wearing, based on another sensor different from the first sensor and the second sensor. The wearing may include the wearable device 103-1 starting to contact the body portion.

In operation 705, the wearable device 103-1 may measure pressure. For example, the wearable device 103-1 may measure the pressure based on at least one sensor. For example, the pressure may include pressure between the wearable device 103-1 and the body portion. For example, the at least one sensor may include the first sensor. Alternatively, for example, the at least one sensor may include a plurality of first sensors, as illustrated in FIG. 4A.

In operation 710, the wearable device 103-1 may identify whether the pressure is less than a first reference pressure. For example, the first reference pressure may be a threshold value for detecting loose wearing of the wearable device 103-1. For example, in case that the pressure is less than the first reference pressure, the wearable device 103-1 may perform operation 715. Alternatively, the wearable device 103-1 may perform operation 720 in case that the pressure is greater than or equal to the first reference pressure.

In operation 715, the wearable device 103-1 may display the first visual object. For example, the first visual object may include information for guiding a change in the loose wearing. For a specific content related to this, a first visual object 771 of FIG. 7B may be referred to.

Referring to an example 770 of FIG. 7B, the wearable device 103-1 may display the first visual object 771. For example, the wearable device 103-1 may display the first visual object 771 for guiding a change from the loose wearing state to a normal wearing state as detecting the loose wearing. For example, the first visual object 771 may include an image and text for guiding that it is the loose wearing. For example, the text may include "a biological signal may not be detected properly, and there is a possibility of a wrist injury due to friction.".

Referring back to FIG. 7A, in operation 720, the wearable device 103-1 may identify whether the pressure is less than a second reference pressure. For example, the second reference pressure may be a threshold value for detecting compression wearing of the wearable device 103-1. For example, in case that the pressure exceeds the second reference pressure, the wearable device 103-1 may perform operation 725. Alternatively, the wearable device 103-1 may perform operation 730 in case that the pressure is less than or equal to the second reference pressure.

In the operation 725, the wearable device 103-1 may display a second visual object. For example, the second visual object may include information for guiding a change in the compression wearing. For a specific content related to this, a second visual object 772 of FIG. 7B may be referred to.

Referring to the example 770 of FIG. 7B, the wearable device 103-1 may display the second visual object 772. For example, the wearable device 103-1 may display the second visual object 772 for guiding a change from the compression wearing state to the normal wearing state as detecting the compression wearing. For example, the second visual object 772 may include an image and text for guiding that it is the compression wearing. For example, the text may include "blood circulation may not be smooth, and pain may be caused by nerve stimulation.".

Although FIG. 7A illustrates that the operation 720 is performed after the operation 710 is performed, the embodiment of the present disclosure is not limited thereto. For example, the wearable device 103-1 may perform the operation 710 and the operation 720 in parallel.

Referring back to FIG. 7A, in the operation 730, the wearable device 103-1 may identify whether a difference between the first pressure value and the second pressure value exceeds a reference difference. For example, in case that the wearable device 103-1 includes a plurality of first sensors in areas, the wearable device 103-1 may identify pressure values for the areas from each of the plurality of first sensors. The wearable device 103-1 may obtain the first pressure value having a minimum pressure and the second pressure value having a maximum pressure among the pressure values. The wearable device 103-1 may recognize whether the difference between the first pressure value and the second pressure value exceeds the reference difference.

In the operation 730, in case that the difference exceeds the reference difference, the wearable device 103-1 may perform operation 735. Alternatively, in the operation 730, in case that the difference is less than or equal to the reference difference, the wearable device 103-1 may perform operation 740.

In the operation 735, the wearable device 103-1 may display a third visual object. For example, in case that the difference exceeds the reference difference, the wearable device 103-1 may display the third visual object. For example, the third visual object may indicate visual information for guiding an unbalance between the pressure values obtained using the plurality of first sensors. For example, the unbalance may be referred to as an unbalance of the wearable device 103-1. For a specific content related to this, a third visual object 783 of FIG. 7C may be referred to.

Referring to an example 780 of FIG. 7C, the wearable device 103-1 may display the third visual object 783. For example, as the wearable device 103-1 detects the unbalance, the wearable device 103-1 may display the third visual object 783 for guiding a change from a unbalance wearing state to the normal wearing state. For example, the third visual object 783 may include an image and text for guiding the unbalance. For example, the text may include "move or press the clock in the direction of the arrow.". The arrow direction may indicate an area where the unbalance is detected of the first sensor in which the first pressure value is obtained.

In the operation 740, the wearable device 103-1 may obtain first data and second data. For example, the wearable device 103-1 may obtain the first data on the pressure between the body portion and the wearable device 103-1 by using the first sensor. The first data may include data obtained for measuring the pressure in the operation 705. However, the embodiment of the present disclosure is not limited thereto. For example, in the operation 740, the wearable device 103-1 may obtain the first data again, by using the first sensor. For example, the first sensor may include the first sensor 303 of FIG. 3A. For example, the first sensor may include at least one of a barometer, a force sensor, or a strain sensor. For example, the first data may be referred to as a sensor value obtained through the first sensor.

For example, the wearable device 103-1 may obtain the second data used to measure the skin state (or condition) of the body portion by using the second sensor. For example, the wearable device 103-1 may obtain the second data used to measure the skin state (or condition) of the body portion, by using the second sensor, while the wearable device 103-1 is worn on the body portion. For example, the skin state (or condition) may include at least one of a temperature, a humidity (or hydration), or active oxygen of the body portion.

For example, the second sensor may include the second sensor 305 of FIG. 3A. For example, the second sensor may include at least one of a sensor for detecting sweat of the body portion, a sensor for detecting the humidity of the body portion, a sensor for detecting the temperature of the body portion, or a sensor for detecting the active oxygen (or antioxidant degree) of the body portion. For example, the second data may be referred to as a sensor value obtained through the second sensor.

In operation 745, the wearable device 103-1 may determine (or predict) whether there is a probability of damage to the skin of the body portion. For example, the wearable device 103-1 may determine (ex. predict) whether there is the probability of damage to the skin, based on a length of time worn on the body portion, the first data, and the second data. For example, the probability of damage may be referred to as degree of damage to the skin, degree of damage prediction of the skin, or state of damage to the skin.

For example, the wearable device 103-1 may obtain a value indicating the degree of damage to the skin, based on the length of time when the wearable device 103-1 is worn on the body portion, the first data, and the second data. For example, the length of the time may indicate a time interval from a timing at which the wearable device 103-1 starts to recognize the wearing on the body portion.

According to an embodiment, the value may be identified based on at least two or more parameters of the temperature, the humidity, the active oxygen, the antioxidant degree, the length of the time, or the pressure. For example, the value may be proportional to the temperature. For example, the value may be proportional to the humidity. For example, the value may be proportional to the active oxygen. For example, the value may be inversely proportional to the antioxidant degree. The value may be proportional to the length of the time. For example, the value may be proportional to a magnitude of the value obtained by subtracting the appropriate pressure from the pressure. For example, the appropriate pressure may indicate a pressure value between the body portion and the wearable device 103-1, when the wearable device 103-1 is in the normal wearing state for the body portion. The normal wearing state may indicate a state in which the wearable device 103-1 is in contact with the body portion and the wearable device 103-1 is supported so as not to be separated from the body portion without external force. For example, as the value increases, the barrier of the skin may be further deteriorated. The value may be referred to as a deterioration level of skin barrier.

According to an embodiment, the value may be identified further based on a skin type. For example, the skin type may include dry, oily, combination, or sensitive of the skin. For example, the information on the skin type may be obtained based on an input of the user. Alternatively, for example, the skin type may be identified by using a statistical model based on a designated number of the second data. For example, the statistical model may use machine learning. For example, the machine learning may be learned based on the designated number of the second data.

According to an embodiment, the wearable device 103-1 may identify whether the value exceeds the reference value. For example, the reference value may be a value for indicating that the barrier of the skin is deteriorated by a designated level.

According to an embodiment, the reference value may be changed according to parameters used to identify the value. For example, in case that parameters used to identify the value are the temperature and the humidity, the reference value may be a first reference value. Alternatively, for example, in case that the parameters used to identify the value are the temperature, the humidity, and the pressure, the reference value may be a second reference value different from the first reference value. Alternatively, for example, in case that the parameters used to identify the values are the active oxygen and the wearing time, the reference value may be a third reference value different from the first reference value and the second reference value.

According to an embodiment, in case that the value is less than or equal to the reference value, the wearable device 103-1 may obtain new first data by using the first sensor and may obtain new second data by using the second sensor.

In operation 750, the wearable device 103-1 may display a fourth visual object. For example, the wearable device 103-1 may display the fourth visual object to guide the taking off (or taking off wearing) of the wearable device 103-1. For example, the wearable device 103-1 may display the fourth visual object to guide the taking off of the wearable device 103-1 on the body portion through the display 307, based on the value exceeding the reference value. For a specific content related to the fourth visual object, FIG. 6B may be referred to.

According to an embodiment, the wearable device 103-1 may control the amount of light used to obtain data through the first sensor or the second sensor while displaying the fourth visual object. For example, the wearable device 103-1 may reduce the amount of light based on the value exceeding the reference value. For example, the amount of light may indicate the amount of light emitted to obtain data through the first sensor or the second sensor.

According to an embodiment, the wearable device 103-1 may provide auditory information or tactile information. For example, the wearable device 103-1 may provide voice information for guiding the taking off, by using a speaker 309 included in the wearable device 103-1. Alternatively, for example, the wearable device 103-1 may provide vibration information for guiding the taking off, by using the actuator 311 included in the wearable device 103-1. The auditory information or the tactile information may be provided together with or separately from the fourth visual object.

In operation 755, the wearable device 103-1 may display a fifth visual object, based on detecting re-wearing in a designated time interval. For example, the wearable device 103-1 may recognize the taking off of the wearable device 103-1 by the user, based on the display of the fourth visual object.

According to an embodiment, the wearable device 103-1 may store a timing at which the taking off starts. For example, the wearable device 103-1 may detect the re-wearing in the designated time interval from the timing at which the taking off starts. For example, the wearable device 103-1 may recognize that the wearable device 103-1 is re-worn on the body portion, based on the at least one sensor included in the wearable device 103-1. For detecting the re-wearing, a content about detecting the wearing of the operation 700 may be applied substantially the same.

According to an embodiment, the wearable device 103-1 may display the fifth visual object for changing the body portion worn by the wearable device 103-1 to another body portion, as detecting the re-wearing in the designated time interval. For a specific content related to this, a fifth visual object 795 of FIG. 7D may be referred to.

Referring to an example 790 of FIG. 7D, the wearable device 103-1 may display the fifth visual object 795. For example, the wearable device 103-1 may display the fifth visual object 795 for changing the body portion to the other body portion, as detecting the re-wearing in the designated time interval. For example, the fifth visual object 795 may include an image and text for guiding wearing on the other body portion. For example, the fifth visual object 795 may include visual information for setting a wearing wrist. For example, in case that the body portion is a right wrist, the other body portion may be a left wrist. For example, in case that the other body portion is selected based on an input of the user for the fifth visual object 795, the wearable device 103-1 may change a display direction of the screen according to the body portion to another display direction according to the other body portion. Alternatively, for example, the fifth visual object 795 may include text guiding to change the body portion to the other body portion.

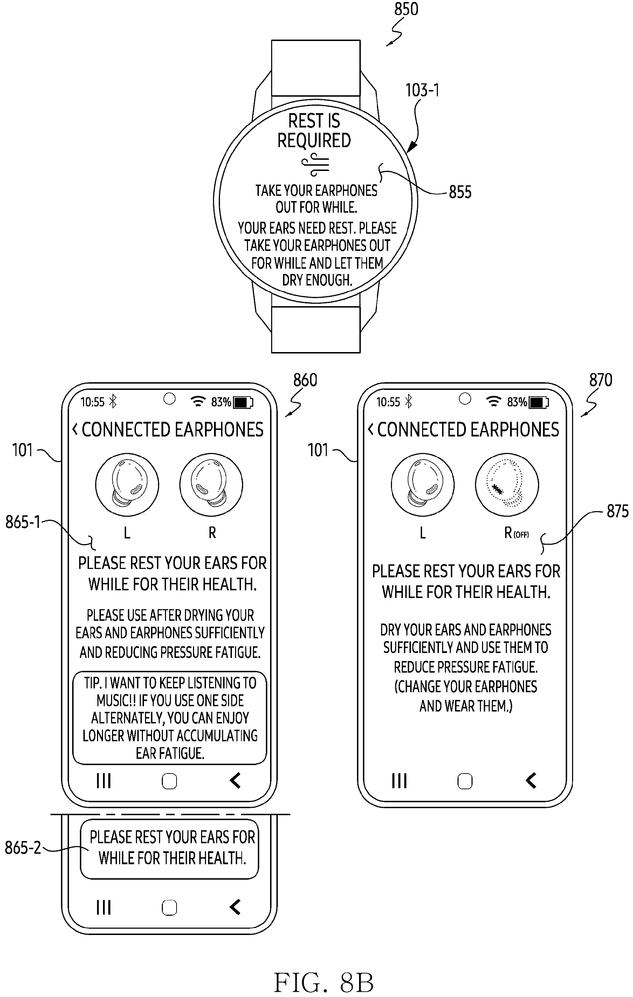

FIG. 8A illustrates an example of an operation flow of a method of recognizing the degree of damage to a body portion on which a wearable device is worn and providing a notification for guiding a wearing method. FIG. 8B illustrates an example of a method of displaying a visual object guiding a wearing method of a wearable device through an external electronic device.

At least a portion of the method of FIG. 8A may be performed by a wearable device 103-2 of FIG. 5A. For example, at least a portion of the method may be controlled by a processor 501 of the wearable device 103-2. In the following embodiment, each operation may be performed sequentially, but is not necessarily performed sequentially. For example, an order of each operation may be changed, and at least two operations may be performed in parallel.

In operation 810, the wearable device 103-2 may obtain first data on movement of the wearable device 103-2, by using the first sensor. For example, the wearable device 103-2 may obtain the first data on the movement, by using the first sensor, based on recognizing that the wearable device 103-2 is worn on the body portion. For example, the body portion may include an ear portion of a user. However, the embodiment of the present disclosure is not limited thereto.

For example, the first sensor may include a first sensor 503 of FIG. 5A. For example, the first sensor may include an accelerometer. For example, the first data may be referred to as a sensor value obtained through the first sensor.

For example, the wearable device 103-2 may recognize that the wearable device 103-2 is worn on the body portion, based on at least one sensor included in the wearable device 103-2. For example, the wearable device 103-2 may recognize the movement of the wearable device 103-2 and may detect the contact between the wearable device 103-2 and the body portion according to amount of change in stimulus, based on the accelerometer.

In operation 820, the wearable device 103-2 may obtain second data used to measure a skin state (or condition) of the body portion, by using the second sensor. For example, the wearable device 103-2 may obtain the second data used to measure the skin state (or condition) of the body portion, by using the second sensor, while the wearable device 103-2 is worn on the body portion. For example, the skin state (or condition) may include at least one of a temperature, a humidity (or hydration), or active oxygen of the body portion.

For example, the second sensor may include a second sensor 505 of FIG. 5A. For example, the second sensor may include at least one of a sensor for detecting sweat of the body portion, a sensor for detecting the humidity of the body portion, a sensor for detecting the temperature of the body portion, or a sensor for detecting the active oxygen (or antioxidant degree) of the body portion. For example, the second data may be referred to as a sensor value obtained through the second sensor.

In operation 830, the wearable device 103-2 may determine (or predict) whether there is a probability of damage to the skin of the body portion. For example, a wearable device 103-2 may determine whether there is the probability of damage to the skin, based on a length of time worn on the body portion, the first data, and the second data. For example, the probability of damage may be referred to as degree of damage to the skin, degree of damage prediction of the skin, or state of damage to the skin.

For example, the wearable device 103-2 may obtain a value indicating the degree of damage to the skin, based on the length of time when the wearable device 103-2 is worn on the body portion, the first data, and the second data. For example, the length of the time may indicate a time interval from a timing at which the wearable device 103-2 starts to recognize the wearing on the body portion.

According to an embodiment, the value may be identified based on at least two or more parameters of the temperature, the humidity, the active oxygen, the antioxidant degree, or the length of the time. For example, the value may be proportional to the temperature. For example, the value may be proportional to the humidity. For example, the value may be proportional to the active oxygen. For example, the value may be inversely proportional to the antioxidant degree. The value may be proportional to the length of the time. In this case, the value may be identified based on the normal wearing state of the wearable device 103-2.

According to an embodiment, it is possible to recognize whether the wearable device 103-2 is in the normal wearing state. The normal wearing state may indicate a state in which the wearable device 103-2 is in contact with the body portion and the wearable device 103-2 is supported so as not to be separated from the body portion without external force. For example, the normal wearing state may be recognized based on the reflection of sound (e.g., chirp or click) for the entire frequency outputted through the speaker 509, a sound difference between the internal microphone and the external microphone of the microphone 507, or a level of physical shielding of the sound played by the speaker 509. For example, the wearable device 103-2 may output sound in the entire frequency band, such as the chirp or the click, through the speaker 509, and may obtain a response curve of the sound reflected through an eardrum in the body portion through the internal microphone. Accordingly, the wearable device 103-2 may recognize the normal wearing state by checking a frequency response curve corresponding to a shape and volume level of an ear canal of the body portion through analysis of each frequency of the obtained sound. Alternatively, for example, the wearable device 103-2 may recognize the normal wearing state, by outputting the sound through the speaker 509 and comparing a magnitude of the sound obtained through each of the internal and external microphones to determine whether the physical shielding level is satisfied. Alternatively, for example, the wearable device 103-2 may recognize the normal wearing state, by checking the contact level between the nozzle 511 and the ear canal, by measuring impedance through the nozzle 511 implemented with the conductive member.

According to an embodiment, the value may be identified further based on a skin type. For example, the skin type may include dry, oily, combination, or sensitive of the skin. For example, the information on the skin type may be obtained based on an input of the user. Alternatively, for example, the skin type may be identified by using a statistical model based on a designated number of the second data. For example, the statistical model may use machine learning. For example, the machine learning may be learned based on the designated number of the second data.

In operation 840, the wearable device 103-2 may provide a notification to guide the taking off wearing (or taking off) of the wearable device 103-2. For example, based on the decision that there is the probability of damage, the wearable device 103-2 may output the notification to guide the taking off of the wearable device 103-2 on the body portion through the speaker 509. Alternatively, for example, the wearable device 103-2, based on the decision that there is the probability of damage (or that there is the probability of occurrence of the damage), may display a visual object indicating the notification through a display of an external electronic device (e.g., an electronic device 101 or the wearable device 103-1 of FIG. 1) connected to the wearable device 103-2. In this case, the wearable device 103-2 may transmit a signal to the external electronic device to cause display of the visual object indicating the notification.

According to an embodiment, the wearable device 103-2 may compare the reference value and the value. For example, the wearable device 103-2 may recognize whether the value exceeds the reference value. For example, the reference value may be a value for indicating that the barrier of the skin is deteriorated by a designated level. For example, in case that the value exceeds the reference value, the wearable device 103-2 may determine that there is the probability of damage.

According to an embodiment, the reference value may be changed according to parameters used to identify the value. For example, in case that parameters used to identify the value are the temperature and the humidity, the reference value may be a first reference value. Alternatively, for example, in case that the parameters used to identify the value are the temperature, the humidity, and the pressure, the reference value may be a second reference value different from the first reference value. Alternatively, for example, in case that the parameters used to identify the values are the active oxygen and the wearing time, the reference value may be a third reference value different from the first reference value and the second reference value.

According to an embodiment, the wearable device 103-2 may control the amount of light used to obtain data through the first sensor or the second sensor while displaying the visual object. For example, the wearable device 103-2 may reduce the amount of light based on the value exceeding the reference value. For example, the amount of light may indicate the amount of light emitted to obtain data through the first sensor or the second sensor.

According to an embodiment, the visual object may include visual information for guiding the taking off of the wearable device 103-2. For a specific example related to this, refer to FIG. 8B below.

FIG. 8B illustrates examples 850, 860, and 870 in which the visual information for guiding the taking off is displayed. For example, in case that the value exceeds the reference value, the wearable device 103-2 may transmit the signal to the external electronic device.

Referring to the example 850, the wearable device 103-1 that received the signal may display a visual object 855 through a display 307 of the wearable device 103-1. For example, the visual object 855 may include an image and text indicating that rest is required. For example, the text may include, "Take your earphones out for a while. Your ears need rest. Please take your earphones out for a while and let them dry enough.".

Referring to the example 860, the electronic device 101 that received the signal may display a visual object 865-1 or 865-2 through a display (e.g., a display module 160) of the electronic device 101. For example, the visual object 865-1 may include the image and text indicating that the rest is required. For example, the visual object 865-2 may include pop-up text. The visual object 865-2 may be superimposed on another visual object (not illustrated).

Referring to the example 870, the electronic device 101 that received the signal may display a visual object 875 through the display (e.g., the display module 160) of the electronic device 101. For example, the visual object 875 may include the image and text indicating that the rest is required. Unlike a visual object 865, the visual object 875 may include an image and text (e.g., R(OFF)) for indicating the release of a portion (e.g., a right part) of the wearable device 103-2. The examples 850, 860, and 870 illustrated in FIG. 8B are only for convenience of explanation, and the embodiments of the present disclosure are not limited thereto.

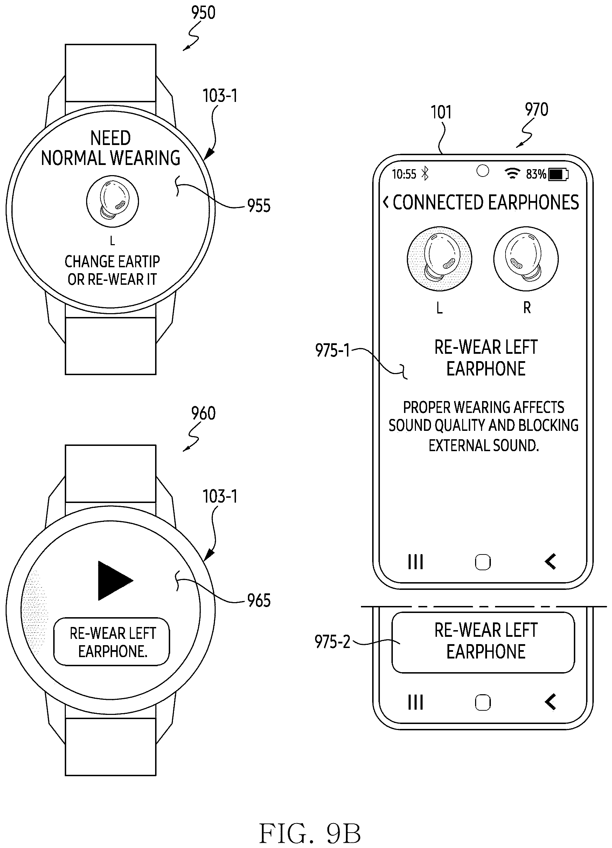

FIG. 9A illustrates an example of an operation flow of a method in which a wearable device worn on an ear portion of a user guides a wearing method. FIG. 9B illustrates an example of a method of displaying a visual object guiding a normal wearing state of a wearable device through an external electronic device.

At least a portion of the method of FIG. 9A may be performed by a wearable device 103-2 of FIG. 5A. For example, at least a portion of the method may be controlled by a processor 501 of the wearable device 103-2. In the following embodiment, each operation may be performed sequentially, but is not necessarily performed sequentially. For example, an order of each operation may be changed, and at least two operations may be performed in parallel.

In operation 900, the wearable device 103-2 may detect wearing. For example, the wearable device 103-2 may recognize that the wearable device 103-2 is worn on the body portion (e.g., the ear portion), based on at least one sensor included in the wearable device 103-2. For example, the wearable device 103-2 may recognize movement of the wearable device 103-2 and may detect contact between the wearable device 103-2 and the body portion according to amount of change in stimulus, based on an accelerometer.

In operation 905, it is possible to recognize whether the wearable device 103-2 is in the normal wearing state. The normal wearing state may indicate a state in which the wearable device 103-2 is in contact with the body portion and the wearable device 103-2 is supported so as not to be separated from the body portion without external force. For example, the normal wearing state may be recognized based on the reflection of sound (e.g., chirp or click) for the entire frequency outputted through the speaker 509, a sound difference between the internal microphone and the external microphone of the microphone 507, or a level of physical shielding of the sound played by the speaker 509.

For example, the wearable device 103-2 may output sound in the entire frequency band, such as the chirp or the click, through the speaker 509, and may obtain a response curve of the sound reflected through an eardrum in the body portion through the internal microphone. Accordingly, the wearable device 103-2 may recognize the normal wearing state by checking a frequency response curve corresponding to a shape and volume level of an ear canal of the body portion through analysis of each frequency of the obtained sound.

For example, the wearable device 103-2 may recognize the normal wearing state, by outputting the sound through the speaker 509 and comparing a magnitude of the sound obtained through each of the internal and external microphones to determine whether the physical shielding level is satisfied.

For example, the wearable device 103-2 may recognize the normal wearing state, by checking a contact level between a nozzle 511 and the ear canal, by measuring impedance through the nozzle 511 implemented with the conductive member.

In operation the 905, the wearable device 103-2 may perform operation 915 in case that it is in the normal wearing state. Alternatively, in the operation 905, the wearable device 103-2 may perform operation 910 in case that it is not in the normal wearing state (i.e., an abnormal wearing state).

In the operation 910, the wearable device 103-2 may provide a notification for guiding the normal wearing state. For example, based on identifying that it is not in the normal wearing state, the wearable device 103-2 may output the notification for guiding the normal wearing state through the speaker 509. Alternatively, for example, the wearable device 103-2 may display a visual object indicating the notification for guiding the normal wearing state through a display of an external electronic device (e.g., an electronic device 101 of FIG. 1 or a wearable device 103-1 of FIG. 2) connected to the wearable device 103-2. In this case, the wearable device 103-2 may transmit a signal to the external electronic device to cause the display of the visual object indicating the notification for guiding the normal wearing state.

According to an embodiment, the visual object indicating the notification may include visual information for guiding the normal wearing state of the wearable device 103-2. For a specific example related to this, refer to FIG. 9B below.

FIG. 9B illustrates examples 950, 960, and 970 in which the visual information for guiding the normal wearing state is displayed. For example, the wearable device 103-2 may transmit the signal to the external electronic device, based on recognizing that it is not in the normal wearing state.

Referring to the example 950, the wearable device 103-1 that received the signal may display a visual object 955 through a display 307 of the wearable device 103-1. For example, the visual object 955 may include an image and text indicating changing the eartip connected to the nozzle 511 or re-wearing the wearable device 103-2. For example, the text may include "Change the eartip or try wearing them again.". In this case, a part of the wearable device 103-2 in which a change is required may be a portion (e.g., a left part) or the whole.

Referring to the example 960, the wearable device 103-1 that received the signal may display a visual object 965 through the display 307 of the wearable device 103-1. For example, the visual object 965 may include an image and text indicating changing the nozzle 511 or re-wearing the wearable device 103-2. For example, the image may include visual information that visually emphasizes the area of the display 307 corresponding to the part (e.g., the left part) of the wearable device 103-2 requiring change. Alternatively, for example, the image may include an animation indicating wearing of the part requiring change.

Referring to the example 970, the electronic device 101 that received the signal may display a visual object 975-1 or 975-2 through a display (e.g., a display module 160) of the electronic device 101. For example, the visual object 975-1 may include the image and text indicating changing the nozzle 511 or re-wearing the wearable device 103-2. For example, the image may include visual information (e.g., shade, color) that visually emphasizes by comparing the part (e.g., the left part) of the wearable device 103-2 requiring change and another part (e.g., the right part). For example, the visual object 975-2 may include the text indicating changing the nozzle 511 or re-wearing the wearable device 103-2. For example, the visual object 975-2 may include pop-up text. The visual object 975-2 may be superimposed on another visual object (not illustrated). The examples 950, 960, and 970 illustrated in FIG. 9B are only for convenience of description, and the embodiments of the present disclosure are not limited thereto.

In the operation 915, the wearable device 103-2 may obtain first data and second data. For example, the wearable device 103-2 may obtain the first data on the movement, by using a first sensor, based on recognizing that the wearable device 103-2 is worn on the body portion. For example, the first sensor may include a first sensor 503 of FIG. 5A. For example, the first sensor may include an accelerometer. For example, the first data may be referred to as a sensor value obtained through the first sensor.

For example, the wearable device 103-2 may obtain second data used to measure a skin state (or condition) of the body portion, by using the second sensor. For example, the wearable device 103-2 may obtain the second data used to measure the skin state (or condition) of the body portion, by using the second sensor, while the wearable device 103-2 is worn on the body portion. For example, the skin state (or condition) may include at least one of a temperature, a humidity (or hydration), or active oxygen of the body portion.

For example, the second sensor may include a second sensor 505 of FIG. 5A. For example, the second sensor may include at least one of a sensor for detecting sweat of the body portion, a sensor for detecting the humidity of the body portion, a sensor for detecting the temperature of the body portion, or a sensor for detecting the active oxygen (or antioxidant degree) of the body portion. For example, the second data may be referred to as a sensor value obtained through the second sensor.

In operation 920, the wearable device 103-2 may determine (or predict) whether there is a probability of damage to the skin of the body portion. For example, a wearable device 103-2 may determine whether there is the probability of damage to the skin, based on a length of time worn on the body portion, the first data, and the second data. For example, the probability of damage may be referred to as degree of damage to the skin, degree of damage prediction of the skin, or state of damage to the skin.

For example, the wearable device 103-2 may obtain a value indicating the degree of damage to the skin, based on the length of time when the wearable device 103-2 is worn on the body portion, the first data, and the second data. For example, the length of the time may indicate a time interval from a timing at which the wearable device 103-2 starts to recognize the wearing on the body portion.

According to an embodiment, the value may be identified based on at least two or more parameters of the temperature, the humidity, the active oxygen, the antioxidant degree, or the length of the time. For example, the value may be proportional to the temperature. For example, the value may be proportional to the humidity. For example, the value may be proportional to the active oxygen. For example, the value may be inversely proportional to the antioxidant degree. The value may be proportional to the length of the time. In this case, the value may be identified based on the normal wearing state of the wearable device 103-2.

According to an embodiment, the value may be identified further based on a skin type. For example, the skin type may include dry, oily, combination, or sensitive of the skin. For example, the information on the skin type may be obtained based on an input of the user. Alternatively, for example, the skin type may be identified by using a statistical model based on a designated number of the second data. For example, the statistical model may use machine learning. For example, the machine learning may be learned based on the designated number of the second data.

According to an embodiment, the wearable device 103-2 may identify whether the value exceeds the reference value. For example, the reference value may be a value for indicating that the barrier of the skin is deteriorated by a designated level.

According to an embodiment, the reference value may be changed according to parameters used to identify the value. For example, in case that parameters used to identify the value are the temperature and the humidity, the reference value may be a first reference value. Alternatively, for example, in case that the parameters used to identify the value are the temperature, the humidity, and the pressure, the reference value may be a second reference value different from the first reference value. Alternatively, for example, in case that the parameters used to identify the values are the active oxygen and the wearing time, the reference value may be a third reference value different from the first reference value and the second reference value.

According to an embodiment, in case that the value is less than or equal to the reference value, the wearable device 103-2 may obtain new first data by using the first sensor and may obtain new second data by using the second sensor.

In operation 925, the wearable device 103-2 may provide a notification for guiding taking off of the wearable device 103-2. For example, the wearable device 103-2 may output, through the speaker 509, the notification for guiding the taking off of the wearable device 103-2 on the body portion, based on the value exceeding the reference value. Alternatively, for example, the wearable device 103-2 may display the visual object indicating the notification, through the display of the external electronic device (e.g., the electronic device 101 of FIG. 1 or the wearable device 103-1 of FIG. 2) connected to the wearable device 103-2. In this case, the wearable device 103-2 may transmit the signal to the external electronic device to cause the display of the visual object indicating the notification.

According to an embodiment, the wearable device 103-2 may control the amount of light used to obtain data through the first sensor or the second sensor while displaying the visual object. For example, the wearable device 103-2 may reduce the amount of light based on the value exceeding the reference value. For example, the amount of light may indicate the amount of light emitted to obtain data through the first sensor or the second sensor.

According to an embodiment, the visual object may include visual information for guiding the taking off of the wearable device 103-2. For example, in case that the value exceeds the reference value, the wearable device 103-2 may transmit the signal to the external electronic device. For a specific example related to this, refer to FIG. 8B described above.

As described above, a wearable device 103-1 comprises a plurality of sensors including a first sensor 303 and a second sensor 305. The wearable device 103-1 comprises memory, including one or more storage mediums, storing instructions. The wearable device 103-1 comprises a display 307. The wearable device 103-1 comprises at least one processor 301 comprising processing circuitry. The instructions, when executed by the at least one processor 301 individually or collectively, cause the wearable device 103-1 to obtain, by using the first sensor 303, first data related to pressure between the wearable device 103-1 and a body portion of a user on which the wearable device 103-1 is worn. The instructions, when executed by the at least one processor 301 individually or collectively, cause the wearable device 103-1 to obtain, by using the second sensor 305, second data used for measuring at least one of a temperature, a humidity, or active oxygen of the body portion while the wearable device 103-1 is worn on the body portion. The instructions, when executed by the at least one processor 301 individually or collectively, cause the wearable device 103-1 to decide whether a probability of damage of a skin of the body portion is happened based on a length of time when the wearable device 103-1 is worn on the body portion, the first data, and the second data. The instructions, when executed by the at least one processor 301 individually or collectively, cause the wearable device 103-1 to display, through the display 307, a visual object to guide taking off the wearable device 103-1 from the body portion based on the decision that the probability of damage is happened. Therefore, the user's skin health may be well preserved in such a way that the wearable device is aware of the user's skin condition and not worn for unduly long time.

According to an embodiment, the instructions, when executed by the at least one processor 301 individually or collectively, may cause the wearable device 103-1 to detect the taking off from the body portion of the wearable device 103-1 by using the first sensor 303. The instructions, when executed by the at least one processor 301 individually or collectively, may cause the wearable device 103-1 to detect re-wearing of the wearable device 103-1 on the body portion within a designated time interval from a timing detected the taking off. The instructions, when executed by the at least one processor 301 individually or collectively, may cause the wearable device 103-1 to display, through the display 307, a visual object to guide wearing the wearable device 103-1 on another body portion, based on detecting re-wearing of the wearable device 103-1.

According to an embodiment, the body portion may include a wrist of the user. The other body portion may include another wrist of the user.

According to an embodiment, the instructions, when executed by the at least one processor 301 individually or collectively, may cause the wearable device 103-1 to detect wearing of the wearable device 103-1 on the body portion by using the first sensor 303. The instructions, when executed by the at least one processor 301 individually or collectively, may cause the wearable device 103-1 to display, through the display 307, a first visual object based on the pressure determined according to the first data less than a first reference pressure. The instructions, when executed by the at least one processor 301 individually or collectively, may cause the wearable device 103-1 to display, through the display 307, a second visual object different from the first visual object based on the pressure greater than or equal to a second reference pressure greater than the first reference pressure. The instructions, when executed by the at least one processor 301 individually or collectively, may cause the wearable device 103-1 to obtain, by using the second sensor 305, the second data in case that the pressure greater than or equal to the first reference pressure and less than the second reference pressure.

According to an embodiment, the first visual object may comprise information on guiding loose wearing of the wearable device 103-1. The second visual object may comprise information on guiding compression wearing of the wearable device 103-1.

According to an embodiment, the first sensor 303 may include at least one of a barometer, a force sensor, or a strain sensor. The second sensor 305 may include at least one of a sensor for detecting sweat of the body portion, a sensor for detecting the humidity of the body portion, a sensor for detecting the temperature of the body portion, or a sensor for detecting the active oxygen of the body portion.

According to an embodiment, the wearable device 103-1 may further include a speaker 309 or an actuator 311. The instructions, when executed by the at least one processor 301 individually or collectively, may cause the wearable device 103-1 to provide sound information through the speaker 309 or vibration information through the actuator 311 together with displaying the visual object.

According to an embodiment, the plurality of sensors may include a plurality of pressure sensor including the first sensor 303 disposed on areas of the wearable device 103-1. The instructions, when executed by the at least one processor 301 individually or collectively, may cause the wearable device 103-1 to obtain a plurality of pressure values for the areas by using the plurality of pressure sensor. The instructions, when executed by the at least one processor 301 individually or collectively, may cause the wearable device 103-1 to display, through the display 307, a visual object for guiding an unbalance of the wearable device 103-1 based on a difference between a first pressure value having a minimum pressure and a second pressure value having a maximum pressure from among the plurality of pressure values greater than a reference gap.

According to an embodiment, the probability of damage of the skin of the body portion may be decided further based on the plurality of pressure values.

According to an embodiment, the instructions, when executed by the at least one processor 301 individually or collectively, may cause the wearable device 103-1 to obtain type information for the skin based on an input of the user. The probability of damage of the skin of the body portion may be decided further based on the type information. The type information may include a dry, an oily, a combination, or a sensitive of the skin.

As described above, a wearable device 103-2 comprises a plurality of sensors including a first sensor 503 and a second sensor 505. The wearable device 103-2 comprises memory, including one or more storage mediums, storing instructions. The wearable device 103-2 comprises a speaker 509. The wearable device 103-2 comprises at least one processor 501 comprising processing circuitry. The instructions, when executed by the at least one processor 501 individually or collectively, cause the wearable device 103-2 to obtain, by using the first sensor 503, first data related to movement of the wearable device 103-2 based on recognizing that the wearable device 103-2 is worn on a body portion of a user. The instructions, when executed by the at least one processor 501 individually or collectively, cause the wearable device 103-2 to obtain, by using the second sensor 505, second data used for measuring at least one of a temperature, a humidity, or active oxygen of the body portion while the wearable device 103-2 is worn on the body portion. The instructions, when executed by the at least one processor 501 individually or collectively, cause the wearable device 103-2 to decide whether a probability of damage of a skin of the body portion is happened based on a length of time when the wearable device 103-2 is worn on the body portion, the first data, and the second data. The instructions, when executed by the at least one processor 501 individually or collectively, cause the wearable device 103-2 to provide, through the speaker 509, a notification to guide taking off the wearable device 103-2 from the body portion based on the decision that the probability of damage is happened. Therefore, the user's skin health may be well preserved in such a way that the wearable device is aware of the user's skin condition and not worn for unduly long time.

According to an embodiment, the body portion may include an ear of the user. The instructions, when executed by the at least one processor 501 individually or collectively, may cause the wearable device 103-2 to recognize the taking off or wearing of the wearable device 103-2. The wearing or the taking off may be identified based on at least one of a frequency analysis for a sound, which is outputted from the speaker 509, obtained through an internal microphone 507-1 of the wearable device 103-2, a comparison for a magnitude of a sound obtained through each of the internal microphone 507-1 and an external microphone 507-2 of the wearable device 103-2, or an impedance measured through a nozzle 511 of the wearable device 103-2.

According to an embodiment, the instructions, when executed by the at least one processor 501 individually or collectively, may cause the wearable device 103-2 to provide, through the speaker 509, a notification to guide not wearing the wearable device 103-2, based on detecting re-wearing of the wearable device 103-2 on the body portion within a designated time interval from a timing recognized the taking off.

According to an embodiment, the first sensor 503 may include an accelerometer. The second sensor 505 may include at least one of a sensor for detecting sweat of the body portion, a sensor for detecting the humidity of the body portion, a sensor for detecting the temperature of the body portion, or a sensor for detecting the active oxygen of the body portion.

According to an embodiment, the wearable device 103-2 may further comprise a communication circuit 513. The instructions, when executed by the at least one processor 501 individually or collectively, may cause the wearable device 103-2 to transmit, to an external electronic device through the communication circuit, information to cause displaying a visual object for the notification on a display of the external electronic device connected through the communication circuit 513. The external electronic device may provide information on a sound outputted on the wearable device 103-2.

According to an embodiment, the instructions, when executed by the at least one processor 501 individually or collectively, may cause the wearable device 103-2 to obtain type information for the skin based on an input of the user. The probability of damage of the skin of the body portion may be decided further based on the type information. The type information may include a dry, an oily, a combination, or a sensitive of the skin.

As described above, in a method performed by a wearable device 103-1, the method may comprise obtaining first data related to pressure between the wearable device 103-1 and a body portion of a user on which the wearable device 103-1 is worn. The method may comprise obtaining second data used for measuring at least one of a temperature, a humidity, or active oxygen of the body portion while the wearable device 103-1 is worn on the body portion. The method may comprise deciding whether a probability of damage to a skin of the body portion is happened based on a length of time when the wearable device 103-1 is worn on the body portion, the first data, and the second data. The method may comprise displaying a visual object to guide taking off the wearable device 103-1 from the body portion based on the decision that the probability of damage is happed.

According to an embodiment, the method may comprise detecting the taking off from the body portion of the wearable device 103-1. The method may comprise detecting re-wearing of the wearable device 103-1 on the body portion within a designated time interval from a timing recognized the taking off. The method may comprise displaying a visual object to guide wearing the wearable device 103-1 on another body portion, based on detecting re-wearing of the wearable device 103-1.

According to an embodiment, the body portion may include a wrist portion of the user. The other body portion may include another wrist portion of the user.

As described above, a method performed by a wearable device 103-2 may comprise obtaining first data related to movement of the wearable device 103-2 based on recognizing that the wearable device 103-2 is worn on a body portion of a user. The method may comprise obtaining second data used for measuring at least one of a temperature, a humidity, or active oxygen of the body portion while the wearable device 103-2 is worn on the body portion. The method may comprise deciding whether a probability of damage to a skin of the body portion is happened based on a length of time when the wearable device 103-2 is worn on the body portion, the first data, and the second data. The method may comprise providing a notification to guide taking off the wearable device 103-2 from the body portion based on the decision that the probability of damage is happed.

As described above, a non-transitory computer-readable storage medium, when individually or collectively executed by at least one processor 301 of a wearable device 103-1 comprising a plurality of sensors including a first sensor 303 and a second sensor 305 and a display 307, may store one or more programs including instructions that cause to obtain, by using the first sensor 303, first data related pressure between the wearable device 103-1 and a body portion of a user on which the wearable device 103-1 is worn. The non-transitory computer-readable storage medium, when individually or collectively executed by the at least one processor 301, may store one or more programs including instructions that cause to obtain, by using the second sensor 305, second data used for measuring at least one of a temperature, a humidity, or active oxygen of the body portion while the wearable device 103-1 is worn on the body portion. The non-transitory computer-readable storage medium, when individually or collectively executed by the at least one processor 301, may store one or more programs including instructions that cause to decide whether a probability of damage of a skin of the body portion is happened based on a length of time when the wearable device 103-1 is worn on the body portion, the first data, and the second data. The non-transitory computer-readable storage medium, when individually or collectively executed by the at least one processor 301, may store one or more programs including instructions that cause to display, through the display 307, a visual object to guide taking off the wearable device 103-1 from the body portion based on the decision that the probability of damage is happened.

As described above, a non-transitory computer-readable storage medium, when individually or collectively executed by at least one processor 501 of a wearable device 103-2 comprising a plurality of sensors including a first sensor 503 and a second sensor 505 and a speaker 509, may store one or more programs including instructions that cause to obtain, by using the first sensor 503, first data related to movement of the wearable device 103-2 based on recognizing that the wearable device 103-2 is worn on a body portion of a user. The non-transitory computer-readable storage medium, when individually or collectively executed by the at least one processor 501, may store one or more programs including instructions that cause to obtain, by using the second sensor 505, a second data used for measuring at least one of a temperature, a humidity, or active oxygen of the body portion while the wearable device 103-2 is worn on the body portion. The non-transitory computer-readable storage medium, when individually or collectively executed by the at least one processor 501, may store one or more programs including instructions that cause to decide whether a probability of damage of a skin of the body portion is happened based on a length of time when the wearable device 103-2 is worn on the body portion, the first data, and the second data. The non-transitory computer-readable storage medium, when individually or collectively executed by the at least one processor 501, may store one or more programs including instructions that cause to provide, through the speaker 509, a notification to guide taking off the wearable device 103-2 from the body portion based on the decision that the probability of damage is happened.

The technical task to be achieved in the present document is not limited to the above-mentioned technical task, and other technical tasks not mentioned will be clearly understood by those who have ordinary knowledge in the technical field belonging to the present document.

The effect that may be obtained in the present disclosure is not limited to the above-mentioned effects, and other effects not mentioned will be clearly understood by those who have ordinary knowledge in the technical field to which the present disclosure belongs.

The electronic device according to various embodiments disclosed herein may be one of various types of electronic devices. The electronic devices may include, for example, a portable communication device (e.g., a smartphone), a computer device, a portable multimedia device, a portable medical device, a camera, a wearable device, or a home appliance. According to an embodiment, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B", "at least one of A and B", "at least one of A or B", "A, B, or C", "at least one of A, B, and C", and "at least one of A, B, or C" may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with", "coupled to", "connected with", or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may be interchangeably used with other terms, for example, "logic", "logic block", "part", or "circuit". A module may be a single integral component, or a minimum unit or part thereof, adapted to perform one or more functions. For example, according to an example, the module may be implemented in a form of an application-specific integrated circuit (ASIC).

Various embodiments as set forth herein may be implemented as software (e.g., a program 140) including one or more instructions that are stored in a storage medium (e.g., an internal memory 136 or an external memory 138) that is readable by a machine (e.g., an electronic device 101). For example, a machine (e.g., a processor 120 of an electronic device 101) of the machine may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., a compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments of the disclosure, each component (e.g., a module or a program) of the above-described components may include a single entity or multiple entities, and some of the multiple entities may be separately disposed in different components. According to various embodiments of the disclosure, one or more of the above-described components may be omitted, or one or more other components may be added. Alternatively or additionally, a plurality of components (e.g., modules or programs) may be integrated into a single component. In such a case, according to various embodiments of the disclosure, the integrated component may still perform one or more functions of each of the plurality of components in the same or similar manner as they are performed by a corresponding one of the plurality of components before the integration. According to various embodiments of the disclosure, operations performed by the module, the program, or another component may be carried out sequentially, in parallel, repeatedly, or heuristically, or one or more of the operations may be executed in a different order or omitted, or one or more other operations may be added.

## Claims

1. A wearable device comprising:
memory, including one or more storage mediums, storing instructions;
a plurality of sensors including a first sensor and a second sensor;
a display; and
at least one processor comprising processing circuitry,
wherein the instructions, when executed by the at least one processor individually or collectively, cause the wearable device to:
obtain (610), by using the first sensor, first data related to pressure between the wearable device and a body portion of a user on which the wearable device is worn;
obtain (620), by using the second sensor, second data used for measuring at least one of a temperature, a humidity, or active oxygen of the body portion while the wearable device is worn on the body portion;
decide (630) whether a probability of damage of a skin of the body portion is happened based on a length of time when the wearable device is worn on the body portion, the first data, and the second data; and
display (640), through the display, a visual object to guide taking off the wearable device from the body portion based on the decision that the probability of damage is happened.

2. The wearable device of claim 1,
wherein the instructions, when executed by the at least one processor individually or collectively, cause the wearable device to:
detect the taking off from the body portion of the wearable device by using the first sensor;
detect re-wearing of the wearable device on the body portion within a designated time interval from a timing detected the taking off; and
display, through the display, a visual object to guide wearing the wearable device on another body portion, based on detecting re-wearing of the wearable device.

3. The wearable device of claim 2,
wherein the body portion includes a wrist of the user;
wherein the other body portion includes another wrist of the user.

4. The wearable device of claim 1 or 2,
wherein the instructions, when executed by the at least one processor individually or collectively, cause the wearable device to:
detect wearing of the wearable device on the body portion by using the first sensor;
display, through the display, a first visual object based on the pressure determined according to the first data less than a first reference pressure;
display, through the display, a second visual object different from the first visual object based on the pressure greater than or equal to a second reference pressure greater than the first reference pressure; and
obtain, by using the second sensor, the second data in case that the pressure greater than or equal to the first reference pressure and less than the second reference pressure.

5. The wearable device of claim 4,
wherein the first visual object comprises information on guiding loose wearing of the wearable device, and
wherein the second visual object comprises information on guiding compression wearing of the wearable device.

6. The wearable device of any of the preceding claims,
wherein the first sensor includes at least one of a barometer, a force sensor, or a strain sensor, and
wherein the second sensor includes at least one of a sensor for detecting sweat of the body portion, a sensor for detecting the humidity of the body portion, a sensor for detecting the temperature of the body portion, or a sensor for detecting the active oxygen of the body portion.

7. The wearable device of claim 1,
wherein the wearable device further includes a speaker or an actuator,
wherein the instructions, when executed by the at least one processor individually or collectively, cause the wearable device to:
provide sound information through the speaker or vibration information through the actuator together with displaying the visual object.

8. The wearable device of any of the preceding claims,
wherein the plurality of sensors includes a plurality of pressure sensors including the first sensor disposed on areas of the wearable device,
wherein the instructions, when executed by the at least one processor individually or collectively, cause the wearable device to:
obtain a plurality of pressure values for the areas by using the plurality of pressure sensor; and
display, through the display, a visual object for guiding an unbalance of the wearable device based on a difference between a first pressure value having a minimum pressure and a second pressure value having a maximum pressure from among the plurality of pressure values greater than a reference gap, and/or
wherein the probability of damage of the skin of the body portion is decided further based on the plurality of pressure values.

9. The wearable device of any of the preceding claims,
wherein the instructions, when executed by the at least one processor individually or collectively, cause the wearable device to:
obtain type information for the skin based on an input of the user,
wherein the probability of damage of the skin of the body portion is decided further based on the type information, and
wherein the type information includes a dry, an oily, a combination, or a sensitive of the skin.

10. A wearable device comprising:
memory, including one or more storage mediums, storing instructions;
a plurality of sensors including a first sensor and a second sensor;
a speaker; and
at least one processor comprising processing circuitry,
wherein the instructions, when executed by the at least one processor individually or collectively, cause the wearable device to:
obtain (810), by using the first sensor, first data related to movement of the wearable device based on recognizing that the wearable device is worn on a body portion of a user;
obtain (820), by using the second sensor, second data used for measuring at least one of a temperature, a humidity, or active oxygen of the body portion while the wearable device is worn on the body portion;
decide (830) whether a probability of damage of a skin of the body portion is happened based on a length of time when the wearable device is worn on the body portion, the first data, and the second data; and
provide (840), through the speaker, a notification to guide taking off the wearable device from the body portion based on the decision that the probability of damage portion is happened.

11. The wearable device of claim 10,
wherein the body portion includes an ear of the user,
wherein the instructions, when executed by the at least one processor individually or collectively, cause the wearable device to:
recognize the taking off or wearing of the wearable device,
wherein the wearing or the taking off is identified based on at least one of:
a frequency analysis for a sound, which is outputted from the speaker, obtained through an internal microphone of the wearable device,
a comparison for a magnitude of a sound obtained through each of the internal microphone and an external microphone of the wearable device, or
an impedance measured through a nozzle of the wearable device.

12. The wearable device of claim 10 or 11,
wherein the instructions, when executed by the at least one processor individually or collectively, cause the wearable device to:
provide, through the speaker, a notification to guide not wearing the wearable device, based on detecting re-wearing of the wearable device on the body portion within a designated time interval from a timing recognized the taking off.

13. The wearable device of any of claims 10 to 12,
wherein the first sensor includes an accelerometer, and
wherein the second sensor includes at least one of a sensor for detecting sweat of the body portion, a sensor for detecting the humidity of the body portion, a sensor for detecting the temperature of the body portion, or a sensor for detecting the active oxygen of the body portion.

14. The wearable device of any of claims 10 to 13,
wherein the wearable device further comprises a communication circuit,
wherein the instructions, when executed by the at least one processor individually or collectively, cause the wearable device to:
transmit, to an external electronic device through the communication circuit, information to cause displaying a visual object for the notification on a display of the external electronic device connected through the communication circuit, the notification providing information on a sound outputted on the wearable device, and/or
wherein the instructions, when executed by the at least one processor individually or collectively, cause the wearable device to:
obtain type information for the skin based on an input of the user,
wherein the probability of damage of the skin of the body portion is decided further based on the type information, and
wherein the type information includes a dry, an oily, a combination, or a sensitive of the skin.

15. A non-transitory computer-readable storage medium, when individually or collectively executed by at least one processor of a wearable device comprising a plurality of sensors including a first sensor and a second sensor and a display, stores one or more programs including instructions that cause to:
obtain, by using the first sensor, first data related pressure between the wearable device and a body portion of a user on which the wearable device is worn;
obtain, by using the second sensor, second data used for measuring at least one of a temperature, a humidity, or active oxygen of the body portion while the wearable device is worn on the body portion;
decide whether a probability of damage of a skin of the body portion is happened based on a length of time when the wearable device is worn on the body portion, the first data, and the second data; and
display, through the display, a visual object to guide taking off the wearable device from the body portion based on the decision that the probability of damage is happened.
